# EUROPEAN PATENT SPECIFICATION

(11) **EP 1 327 424 B1**
(45) Date of publication and mention of the grant of the patent: **12.09.2012**
(21) Application number: 03000744.7
(22) Date of filing: 13.01.2003
(51) Int. Cl.: A61F 2/38

(54) **Surgically implantable knee prosthesis having medially shifted tibial surface**
Knieendoprothese mit medial versetzter Tibiafläche
Endoprothèse du genou à surface tibiale déplacée médialement

(30) Priority: 11.01.2002 US 44756; 11.01.2002 US 44224; 11.01.2002 US 44677; 11.01.2002 US 44159
(43) Date of publication of application: 16.07.2003
(73) Proprietor: Fell, Barry M., Hummelstown, PA 17036 (US); Hallock, Richard H., Hummelstown, PA 17036 (US)
(72) Inventor: Fell, Barry M., Hummelstown, Pennsylvania 17036 (US); Hallock, Richard H., Hummelstown, Pennsylvania 17036 (US); Wheeler, John L., Round Rock, Texas 78681 (US)
(74) Representative: Miller, James Lionel Woolverton

(56) References cited:
- EP-A- 0 372 811
- EP-A- 1 097 679
- WO-A-95/14444
- FR-A- 2 736 819
- FR-A- 2 747 914
- FR-A- 2 778 332
- US-A- 4 340 978
- US-B1- 6 206 927

## Description

The present invention pertains to prosthetic devices. More particularly, the invention pertains to knee joint prostheses which may be surgically implanted between the femoral condyle and tibial plateau of the knee joint.

Articular cartilage and meniscal cartilage provide the mobile weight bearing surfaces of the knee joint. Damage to these surfaces is generally due to genetic predisposition, trauma, and/or aging. The result is usually the development of chondromalacia, thinning and softening of the articular cartilage, and degenerative tearing of the meniscal cartilage. Various methods of treatment are available to treat these disease processes. Each option usually has specific indications and is accompanied by a list of benefits and deficiencies that may be compared to other options.

The healthy knee joint has a balanced amount of joint cartilage across the four surfaces of this bi-compartmental joint (medial femoral condyle, medial tibial plateau, lateral femoral condyle and lateral tibial plateau). In patients with osteoarthritis, degenerative process typically leads to an asymmetric wear pattern that leaves one compartment with significantly less articular cartilage covering the distal portions (or weight bearing area) of the tibia and femur than the other compartment. Most commonly, the medial compartment of the knee joint is affected more often than the lateral compartment.

As the disease progresses, large amounts of articular cartilage are worn away. Due to the asymmetric nature of the erosion, the alignment of the mechanical axis of rotation of the femur relative to the tibia becomes tilted down towards the compartment which is suffering the majority of the erosion. The result is a Varus (bow-legged) deformity in the case of a medial compartment disease predominance, or a Valgus (knock-kneed) deformity in the case of lateral compartment disease predominance. Factors such as excessive body weight, previous traumatic injury, knee instability, the absence of the meniscus and genetic predisposition, all affect the rate of the disease.

The disease is usually defined in stages of Grade I through V, with Grade III revealing significant articular cartilage loss, Grade IV revealing some eburnation of the subchondral bone, and Grade V detailing both significant articular loss and bone loss.

It is important to understand that the disease manifests itself as periodic to continuous pain that can be quite uncomfortable for the patient. The cause of this pain is subject to many opinions but it is apparent that, as the joint compartment collapses, the collateral ligament on the side of the predominant disease becomes increasingly slack (like one side of a pair of loose suspenders), and the tibial and femoral axes move, for example, from a Varus to a Valgus condition. This increases the stress on the opposing collateral ligament (and cruciate ligaments as well) and shifts the load bearing function of this bi-compartmental joint increasingly towards the diseased side. This increasing joint laxity is suspected of causing some of the pain one feels. In addition, as the bearing loads are shifted, the body responds to the increased loading on the diseased compartment with an increased production of bony surface area (osteophytes) in an attempt to reduce the ever-increasing areal unit loading. All of this shifting of the knee component geometry causes a misalignment of the mechanical axis of the joint. The misalignment causes an increase in the rate of degenerative change to the diseased joint surfaces causing an ever-increasing amount of cartilage debris to build up in the joint, further causing joint inflammation and subsequent pain.

Currently, there is a void in options used to treat the relatively young patient with moderate to severe chondromalacia involving mainly one compartment of the knee. Current treatments include NSAIDs, cortisone injections, hyaluronic acid (HA) injections and arthroscopic debridement. Some patients cannot tolerate or do not want the risk of potential side effects of NSAIDs. Repeated cortisone injections actually weaken articular cartilage after a long period of time. HA has shown promising results but is only a short term solution for pain. Arthroscopic debridement alone frequently does not provide long lasting relief of symptoms. Unfortunately, the lack of long term success of these treatments leads to more invasive treatment methods. Osteochondral allografts and microfracture techniques are indicated for small cartilage defects that are typically the result of trauma. These procedures are not suitable for addressing large areas of degeneration. In addition, osteochondral allografts can only be used to address defects on the femoral condyle. Tibial degeneration can not be addressed with this technique. High tibial osteotomy (HTO) corrects the varus malalignment between the tibia and femur but, because it is performed below the joint line, it does not fill the cartilage void or retension the medial collateral ligament (MCL). Removing bone and changing the joint line does not complicate the conversion to total knee arthroscopy (TKA). However, an HTO does leave a hard sclerotic region of bone which is difficult to penetrate making conversion to a total knee replacement (TKR) technically challenging. Unicompartmental and bicompartmental total knee replacements resect significant amounts of bone and, if performed on younger patients, will likely require revision surgery as they age. Revision total knee replacement surgery is usually extensive and results in predictably diminished mechanical life expectancy. Therefore, it is best to delay this type of bone resecting surgery as long as possible.

The only true solution is to rebuild the defective joint by "filling" the joint space with more articular bearing material through a complete resurfacing of the existing femoral condyle and tibial plateau. By replacing the original cartilage to its pre-diseased depth, the joint mechanical axis alignment is restored to its original condition. Unfortunately, these natural articular materials and surgical technology required to accomplish this replacement task do not yet exist.

Currently, replacement of the existing surfaces, with materials other than articular cartilage, is only possible with a total or uni-condylar knee replacement, and these procedures require removal of significant amounts of the underlying bone structure.

The alternative method is to fill the joint space with a spacer that replaces the missing articular materials. This spacer should also provide an anatomically correct bearing surface for both the tibial and femoral surface (U.S. Patent 6,206,927).

Attaching a new bearing surface to the femoral condyle is technically challenging and was first attempted, with limited success, over 40 years ago with the MGH (Massachusetts General Hospital) knee. Like a dental crown, it covered both femoral condyles with Vitallium (CoCr) and would bear against the existing tibial plateau, as it is known for example from WO 95/14444 A.

Tibial covering devices such as the McKeever, Macintosh and Townley tibial tray, maintained the existing femoral surface as the bearing surface, but like the MGH knee, all required significant bone resection, thus making them less than ideal solutions as well.

These devices also made no particular attempt to match the patient's specific femoral or tibial geometry thus reducing their chances for optimal success. Because these devices were made of CoCr, which has different visco-elastic and wear properties from the natural articular materials, any surface geometry which did not closely match the bearing surface of the tibia or femur, could cause premature wear of the remaining cartilage due to asymmetric loading.

Newer materials technologies in development include filling the joint space by injecting polyurethane (U.S. Patent 5,795,353) into the joint and anchoring it with holes drilled into the tibial plateau. Others include a series of polymeric materials such as PVA Hydrogels in a titanium mesh as described by Chang et al, "Historical Comparison of Tibial Articular Surfaces Against Rigid Materials And Artificial Articular Cartilage," Journal of Biomedical Material Research, 37, 51-59, 1997, biodegradable anhydride prepolymers that can be cross linked with irradiation by UV light (U.S. Patent 5,902,599) and in-vivo grown articular chondrocytes in a collagen fiber or other bio-compatible scaffold (U.S. Patent 5,158,574). Other low surface energy materials, such as low temperature isotropic (LTI) pyrolitic carbon, have been investigated as bearing surfaces as well.

A knee implant comprising the features of the preamble of claim 1 is known from EP 372 811 A1.

All of these techniques are limited by one's ability to first of all fashion these materials in a conformal fashion to replicate the existing knee geometry, while at the same time, maintaining their location within the joint while further being able to survive the mechanical loading conditions of the knee.

Therefore, what is needed is a uni-compartmental interpositional spacer which, by effectively replacing worn articular material, restores normal joint alignment without requiring any bone resection or any means of bone fixation and provides an anatomically correct bearing surface for the femoral condyle to articulate against.

This is solved by the present invention which is defined by claim 1.

According to one embodiment, an implantable knee prosthesis includes a body having a substantially elliptical shape in plan and a pair of opposed faces. A peripheral edge of variable thickness extends between the faces and includes a first side, a second side opposite the first side, a first end and a second end opposite the first end. The thickness of the peripheral edge at the first side is greater than the thickness of the peripheral edge at the second side, the first end and the second end.

According to another embodiment, the prosthesis includes a body having a substantially elliptical shape in plan and a pair of opposed faces; a first one of the faces has a first shape; a second one of the faces has a second shape different from the first shape; and the peripheral edge extending at an incline between the first face and the second face.

According to another embodiment, the prosthesis includes a body having a substantially elliptical shape in plan and including a first portion and second portion; the first portion having a first peripheral edge and a first face; the second portion having a second peripheral edge and a second face; and the second peripheral edge extending radially outwardly from the first peripheral edge to form a shoulder between the first portion and the second portion.

According to another embodiment, the prosthesis comprises a two-piece body having a substantially elliptical shape in plan; a first piece being a tibial piece including a tibial surface; a second piece being a femoral piece including a femoral surface; the first piece and the second piece being mutually slidably engagable and separable.

The invention may be used in a method of providing a knee prosthesis comprising: providing a body having a substantially elliptical shape in plan and a pair of opposed faces; extending a peripheral edge of variable thickness between the faces, the peripheral edge having a first side, a second side opposite the first side, a first end and a second end opposite the first end; and providing the thickness of the peripheral edge at the first side to be greater than the thickness of the peripheral edge at the second side, the first end and the second end.

In another example the method comprises the steps of: providing a body having a substantially elliptical shape in plan and a pair of opposed faces; providing a first one of the faces with a first shape; providing a second one of the faces with a second shape, different from the first shape; and providing a peripheral edge extending at an incline between the first face and the second face.

In another example the method comprises the steps of: providing a body having a substantially elliptical shape in plan and including a first portion and a second portion; providing the first portion with a first peripheral edge and a first face; providing the second portion with a second peripheral edge and a second face; and extending the second peripheral edge radially outwardly from the first peripheral edge to form a shoulder between the first portion and the second portion.

In another example the method comprises the steps of: providing a two-piece body having a substantially elliptical shape in plan and including a first piece and a second piece; providing the first piece with a tibial surface; providing the second piece with a femoral surface; and the first piece and the second piece being mutually slidably engagable and separable.

The invention will be described in detail in the following drawings, in which:
- Fig. 1: is a plan view illustrating an embodiment of an implantable knee prosthesis.
- Fig. 2: is a cross-sectional view taken along the line A-A of Fig.1.
- Fig. 3: is a cross-sectional view taken along line B-B of Fig. 1.
- Figs. 4a-4e: illustrate several views of an embodiment of the device.
- Figs. 5a-5d: illustrate several views of another exemplary device.
- Figs. 6a-6d: illustrate several views of an exemplary device.
- Figs. 7a-7d: illustrate several views of another exemplary device.
- Figs. 8a-8d: illustrate several views of an exemplary device.
- Figs. 9a-9d: illustrate several views of another example of a device.
- Figs. 10a-10e: illustrate several views of an exemplary device.
- Figs. 11a-11d: illustrate several views of another exemplary device.
- Figs. 12a-12d: illustrate several views of a further examples of a device.
- Fig. 13: illustrates placement of the prosthesis in a knee joint.

The present device is an implantable knee prosthesis in the form of a uni-compartmental interpositional spacer which, by effectively replacing worn articular material, restores the normal joint alignment and provides a congruent bearing surface for the femoral condyle to articulate against. Further, it essentially eliminates articulation against the tibial surface thereby preventing further degradation of the tibial surface. Degeneration of the femoral anatomy is significantly reduced because the conforming femoral surface of the device accommodates the complex shape of the femoral condyle in extension as well as in flexion. Insertion of the device is done via a 3 cm to 5 cm medial parapatella incision after arthroscopic debridement of the femoral and tibial cartilage and removal of medial meniscus toward the rim along the anterior, medial and posterior portions. No bone resection or mechanical fixation of the device is required. Only osteophytes which interfere with the device placement or with proper collateral ligament alignment are removed. The device is offered in multiple thicknesses in order to occupy the joint space and tighten the "loose suspenders" problem of the collateral ligaments. By occupying the joint space and retensioning the collateral ligaments, the unicompartmental interpositional spacer improves joint stability and restores the limb to a more normal mechanical alignment.

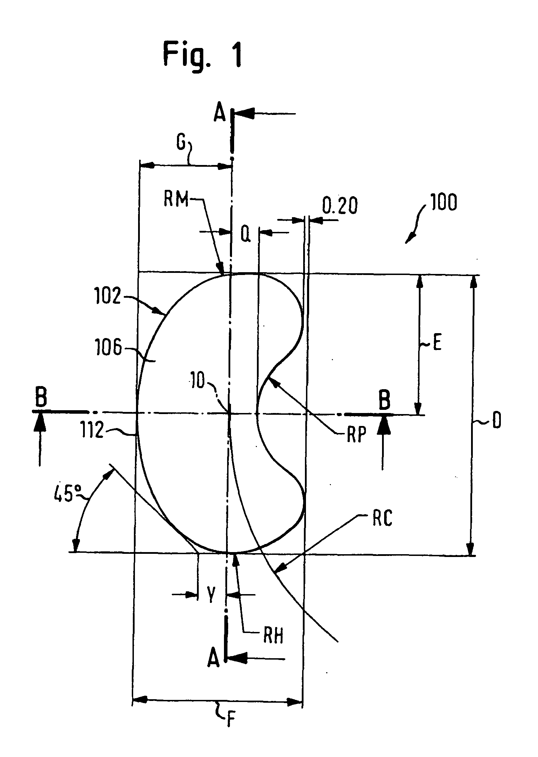

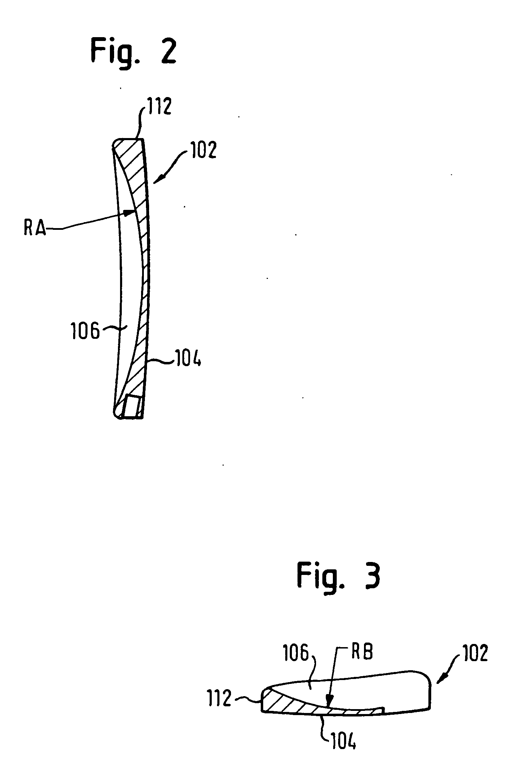

An implantable knee prosthesis 100 is illustrated in Fig. 1. An anterior/posterior (A/P) cross-sectional view is taken along section line A-A and illustrated in Fig. 2. A medial/lateral (M/L) cross-sectional view is taken along section line B-B and illustrated in Fig. 3. A Coordinate System Origin (CSO) 10 is at the intersection of lines A-A and B-B. Prosthesis 100, Figs. 1-3, includes a body 102 having a peripheral edge 112, a first or tibial face 104 and a second or femoral face 106.

The current mechanical structure is a compromise between the geometry of the femoral condyle and the kinematics of the knee. Specifically, the femoral condyle has two major AP radii such that when the knee is in full extension, one radius position is in contact with the tibial plateau while, during flexion, another portion of the femoral condyle is in contact with the tibial plateau. Further complications arise when it is recognized that the femur rotates with respect to the tibia during flexion, thereby changing the orientation of the femoral anatomy to the tibial plateau. Much study has been dedicated to determine if any relationship exists in the normal human anatomy that would allow one to define the required dimensions of the device for proper fit and function based on a single, easy to establish, measurable anatomic landmark. Based on a study of over 100 MRI's and 75 X-rays of human subjects ranging from 15 to 87 years of age, a relationship was established between the anteroposterior radius of the most distal portion of the femoral condyle and the dimensions which control the geometric form of the device. The database revealed a range of femoral anteroposterior radii from 32mm to 48mm. However, it is known that the worldwide range is much larger because of race differences in the human anatomy.

A preferred method of construction aligns the apex of a femoral radius with the CSO 10. Fig. 1. The apex of a tibial surface is also generally aligned in both the A/P and M/L directions with the CSO 10, but is separated vertically from the CSO 10 to create the part thickness. The substantially oval shape of the peripheral edge 112 is then located with respect to the CSO 10. In general, the CSO 10 of the device is located at the center of the ellipse and a minor axis of the ellipse F is related to a major axis D by a ratio ranging from F=.25D to 1.5D with a preferred value of =.64D. Similar ratios can be established for all of the controlling dimensions of the part such that the shape in plan, i.e., as observed from above or below, femoral surface geometry, and tibial surface geometry for a normal tibial anatomy can generally be defined by one physical A/P measurement of the patient's tibial anatomy. The appropriate thickness of the implant can be determined by measuring the amount of joint space between the femoral and tibial surface when a minor amount of valgus (heels out, knees in) is applied to the knee.

Referring again to Figs. 1-3, the preferred relationship between femoral radius RA to other joint dimensions (femoral radius is the driving radius to all other dimensions) is as follows:
- Medial-lateral radius RB = .25RA to 1.0RA
- Curve of anterior half of Femoral Radius RC = .5RA to 2.0RA, Posterior half is straight
- Length D = .6RA to 1.4RA
- Posterior half E = .1RA to .75RA
- Width F = .25RA to 1.5RA
- Width from part center to medial edge G = .096RA to .48RA
- Anterior plan radius RH = .16RA to .64RA
- Posterior plan radius RM = .16RA to .64RA
- Radius along lateral spine area RP =.1RA to 2.0RA
- Width from part center to lateral edge Q = -.32RA to +.32RA
- Location of transition from anterior radius to medial radius Y = -.32RA to +.32RA
(a negative value means that a dimension may extend to an opposite side of section line A-A).

Below are the preferred ratios used to define the shape of prosthesis 100 in terms of the dimension RA, i.e. the femoral radius of prosthesis 100.
RP = .238RA
E = .5RA
F = .64RA
RH = .32RA
M = .384A
G = .352RA
Q = .1056RA
Y = .1152RA
R B = .68RA
D = RA
RC = RA

The actual shape of the present device may be tailored to the individual. Individuals with high varus or valgus deformation due to wear, degeneration, or disease, may require a device which is of considerably greater thickness over the portions where wear is most advanced. For example, many patients who suffer from this early stage of degenerative arthritis will have large areas of eburnated bone along the medial edge of the tibial plateau and femoral condyle but have significant cartilage remaining along the tibial spine. In these instances the tibial surface of the implant may be thicker along the medial edge to accommodate the defects on the tibial plateau and enhance the stability of the device. An implant made to these specifications would be more wedge-shaped when viewed in a frontal plane, with the medial side of the implant being the larger side of the wedge. This wedge could be oriented in any direction to accommodate the specific location of significant cartilage loss for a given patient.

Alternatively, the cartilage loss can be concentrated in the central load bearing portion of the femoral condyle. This condition results in a femoral condyle which is essentially flat when the knee is in terminal extension. In order to bridge the flattened area of the femoral condyle, the femoral surface of a specific implant size can be enlarged while maintaining the geometric area of the tibial surface. This modification of the implant would prevent overhang of the tibial surface beyond the border of the tibial plateau while providing a larger surface area to distribute the contact loads at the femoral surface. In other instances, it may be preferable to decrease the femoral surface area for a given implant size.

Degeneration in the medial compartment will cause the femoral condyle to shift towards the medial edge of the tibia such that the center of the femur is no longer directly above the center of the tibia. In some patients it may be desirable to offset the femoral surface of the implant laterally with respect to-the tibial geometry to put the femur back in a more normal alignment. Other degenerative conditions can exist which could be accommodated by offsetting and/or rotating the femoral geometry in a variety of directions with respect to the tibial surface.

In youthful patients, where trauma-induced damage rather than severe wear or degeneration has occurred, differences in device thickness will be more moderate. In general, the device is kidney-shaped when viewed in plan, and has a negative meniscus shape when viewed from the side, i.e.; the thickness along the periphery of the device being greater than the thickness along the center of the device. The kidney-shape in plan may be described generally as elliptical, the shape resembling a distorted ellipse.

One skilled in the art can approximate the generally elliptical shape with a combination of straight lines and radial blends. Therefore, the term "substantially elliptical" is intended to include all construction methods which yield a planar shape which is longer in one direction than the transverse direction, and has rounded corners.

The present invention is intended to fill in the space that results from cartilage loss on both the femoral condyle and tibial plateau. The thickness of the implant at the CSO should be approximately equal to the combined amount of cartilage loss from the two boney surfaces. When an implant of proper thickness is inserted between the femur and the tibia, the limb is restored to its proper anatomic alignment and ligament structures around the knee are retensioned.

As previously described, the implant is thicker at the posterior edge than at the CSO because it replicates the shape of the intact meniscus. In order for the implant to center itself on the surface of the tibia, the thick posterior edge of the device must be forced beyond the most distal aspect of the femur where the space between the femur and tibia is the smallest. Insertion of the implant is accomplished by forcing the medial compartment joint space open while lifting the tibia over the posterior edge of the implant. To make the insertion of the implant easier, the implant could be separated into a femoral portion and a tibial portion. The femoral portion could be positioned against the distal femur and then the tibial portion could be inserted into the knee separately. The two portions could engage each other along a linear or curved runner to insure proper orientation between the articulation surfaces. The runner would preferably provide a slidable connection, such as a dovetail, between the two portions that would prevent them from separating.

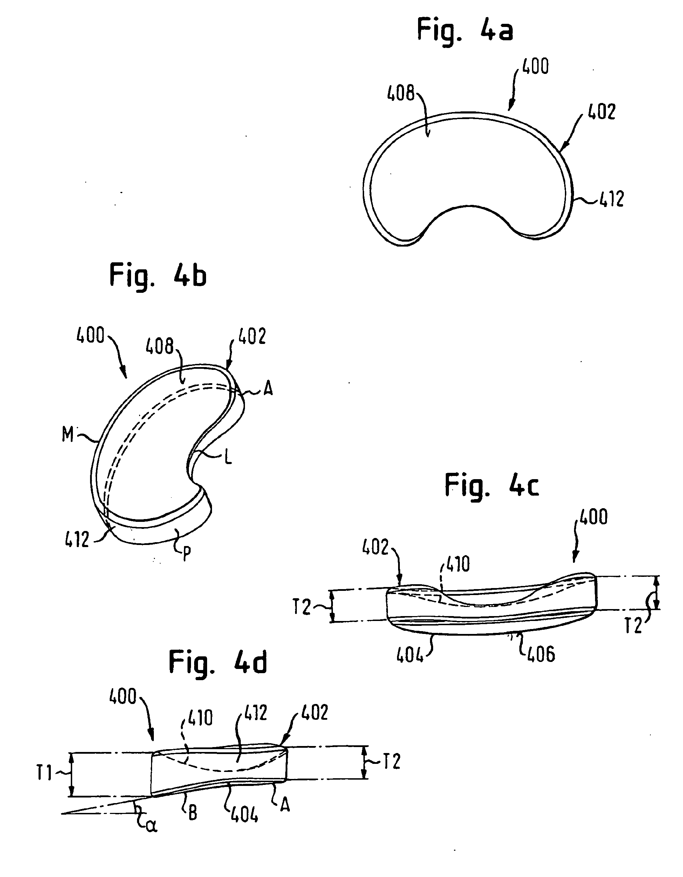

For example, in the embodiments of Figs. 4a-4d, an implantable knee prosthesis is generally designated 400 and includes a body 402 having a substantially elliptical shape in plan, and a pair of opposed faces. A first or tibial face 404 includes a convex surface 406. A second or femoral face 408 includes a concave surface 410. More specifically, first face 404 and second face 408 are substantially kidney shaped:

A peripheral edge 412 of variable thickness extends between the first face 404 and the second face 408. The peripheral edge 412 includes a first or medial side M, a second or lateral side L, opposite the first side M, a first or anterior end A, and a second or posterior end P, opposite the first end A.

The first side M of the peripheral edge 412 is of a first thickness T1. The second side L, the first end A, and the second end P, each have a second thickness T2, which is less than T1. According to the invention, the difference between first thickness T1 and second thickness T2 is accomplished by providing the first face 404 with a first portion A and a second portion B. The second portion B extends at an angle α relative to the first portion A. The additional thickness of T2 is thus medially shifted on the tibial face 404 to accommodate bone loss.

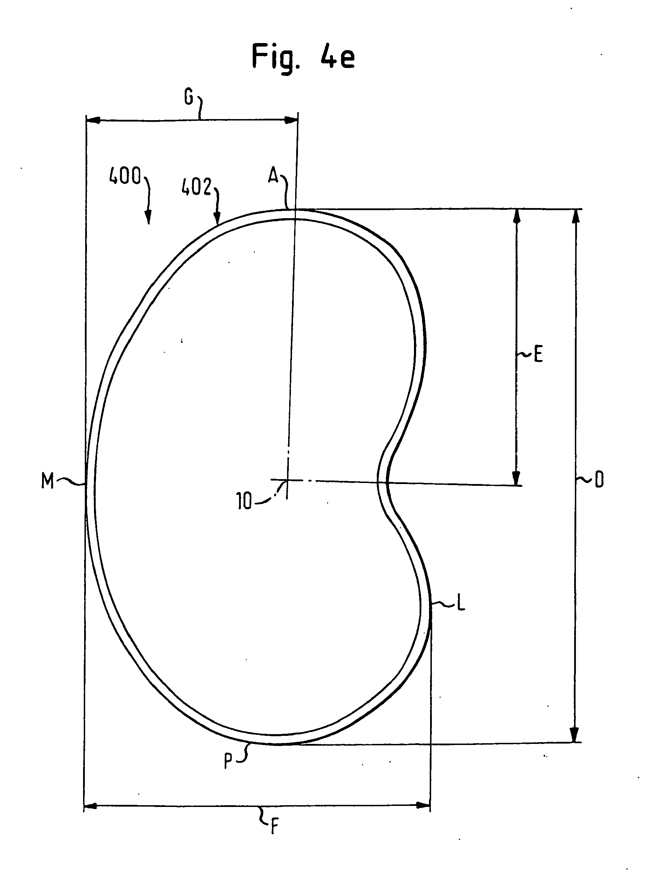

Prosthesis 400 as viewed in Fig. 4e, is similar to prosthesis 100, Fig. 1, in that a dimension D, in a range of from about .6A to about 1.4A, is defined by the first end A and the second end P. Another dimension F is defined by the first side M and the second side L. It has been found that a suitable size for body 402 is defined by the dimension F being from about .25A to about 1.5A of the dimension D. Also a dimension E extends from the CSO 10 of the body 402 to the first end A. It has also been found that a suitable size for body 402 is also defined by the dimension E being from about .1A to about .75A of the dimension D. Further, a dimension G extends substantially from the CSO 10 of body 402 to the first side M. It has further been found that a suitable size for body 402 is further defined by the dimension G being from about .096A to about .48A of the dimension F.

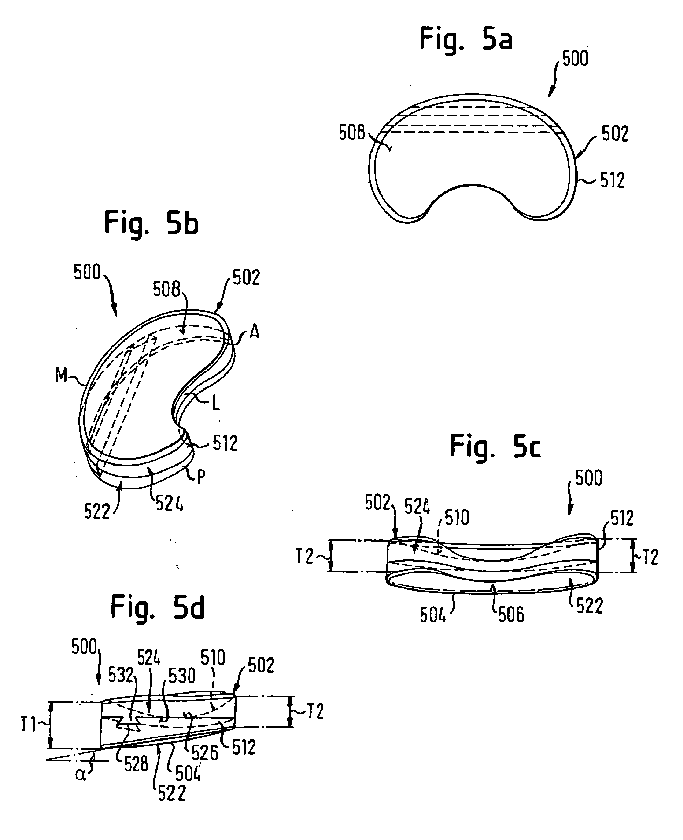

In another example, Figs. 5a-5d disclose an implantable knee prosthesis generally designated 500 including a body 502 having a substantially elliptical shape in plan, and a pair of opposed faces. A first or tibial face 504 includes a convex surface 506. A second or femoral face 508 includes a concave surface 510. More specifically, first face 504 and second face 508 are substantially kidney shaped.

A peripheral edge 512 of variable thickness extends between the first face 504 and the second face 508. The peripheral edge 512 includes a first or medial side M, a second or lateral side L, opposite the first side M, a first or anterior end A, and a second or posterior end P, opposite the first end A.

The first side M of the peripheral edge 512 is of a first thickness T1. The second side L, the first end A and the second end P, each have second thickness T2, which is less than T1. The difference between the first thickness T1 and the second thickness T2 may be accomplished, for example by providing a first body piece 522 attached to a second body piece 524. The first body piece 522 includes the first face 504 and the second body piece 524 includes the second face 508. The first face 504 is at an angle α relative to the second face 508.

The first body piece 522 may also have a keyed sliding interconnection with the second body piece 524. For example, a surface 526 of the first body piece 522 may include a keyway 528, and a surface 530 of the second body piece 524 may include a key 532 for sliding engagement with keyway 528 at an interface of the respective surfaces 526 and 530. Thus, the additional thickness T2 is medially shifted on the tibial face 504 to accommodate bone loss.

In the embodiments of Figs. 4a-4d and the example of Figs. 5a-5d, the convex surface of the first face includes a contour angle (discussed above) which is substantially the same as an associated contour angle of a tibial plateau. The concave surface of the second face includes a contour angle (discussed above) which is substantially the same as an associated femoral condyle. In this manner, the first and second faces are contoured such that the prosthesis is self-centering between a tibial plateau and a femoral condyle as discussed above.

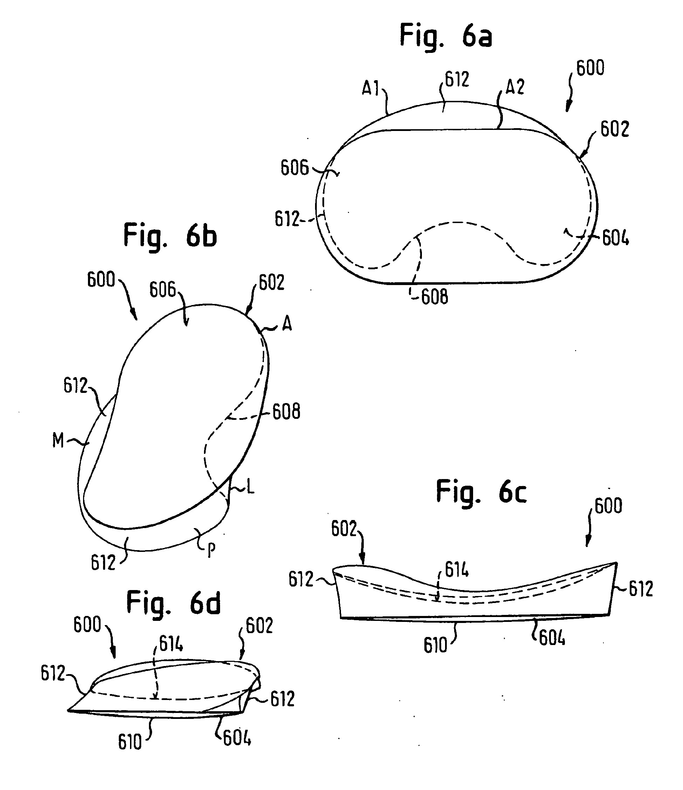

In some patients, it may be beneficial to provide an insert different tibial and femoral surface profiles. For example, in one of the examples of Figs. 6a-6d, an implantable knee prosthesis 600 comprises a body 602 having a generally elliptical shape in plan including a first face 604 and a second face 606. The first face 604 is substantially kidney shaped in plan and the second face 606 is substantially elliptical in plan. The first face 604 preferably includes a notch 608 and a convex surface 610. The second face 606 includes a concave surface 614.

A peripheral edge 612 extends at an incline between the first face 604 and the second face 606. As a result, the second face 606 is different in shape, and laterally offset so as to extend over the notch 608 of the first face 604. The first face 604 has an area A1 and the second face 606 has an area A2, different than the first area A1.

The peripheral edge 612 includes a first or medial side M, a second or lateral side L, opposite the first side M, a first or anterior end A and a second or posterior end P opposite the first end A.

The first face 604 includes a contour which is substantially the same as an associated contour of a tibial plateau. The second face 606 includes a contour which is substantially the same as an associated contour of a femoral condyle. In this manner, the first face 604 and the second face 606 are contoured such that the prosthesis 600 is self-centering between a tibial plateau and a femoral condyle as discussed above.

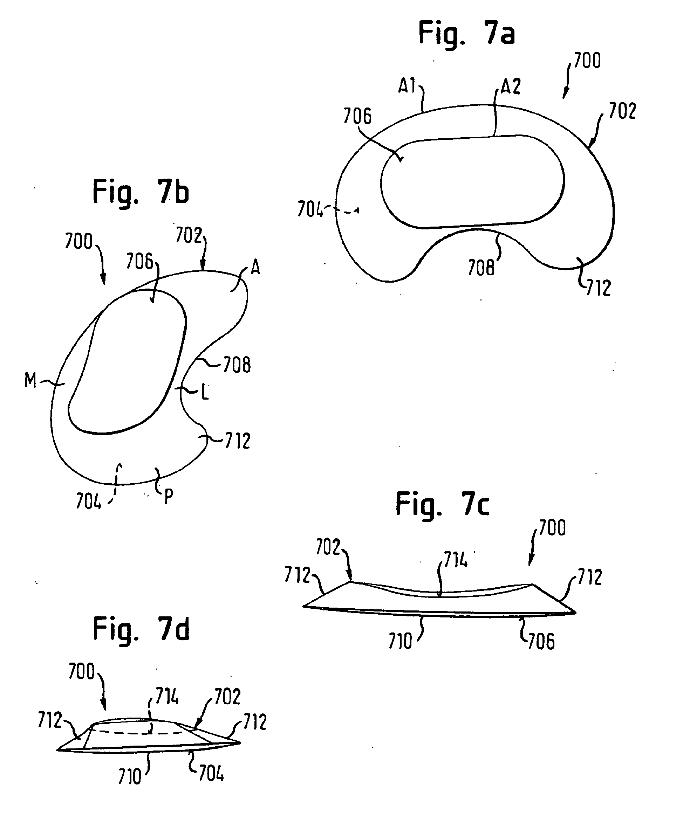

In another one of the examples of Figs. 7a-7d, an implantable knee prosthesis 700 comprises a body 702 having a generally elliptical shape in plan including a first face 704 and a second face 706. The first face 704 is substantially kidney shaped in plan and the second face 706 is substantially elliptical in plan. The first face 704 preferably includes a notch 708 and a convex surface 710. The second face 706 includes concave surface 714.

A peripheral edge 712 extends to form a converging incline between the first face 704 and the second face 706. As a result, the first face 704 has a first area A1 and the second face 706 has a second area A2, different in shape, and smaller than the first area A1. The peripheral edge 712 also includes opposed medial and lateral sides, M and L, respectively, and anterior and posterior ends, A and P, respectively.

The first face 704 includes a contour which is substantially the same as an associated contour of a tibial plateau. The second face 706 includes a contour which is substantially the same as an associated contour of a femoral condyle. In this manner, the first face 704 and the second face 706 are contoured such that the prosthesis 700 is self-centering between a tibial plateau and a femoral condyle as discussed above.

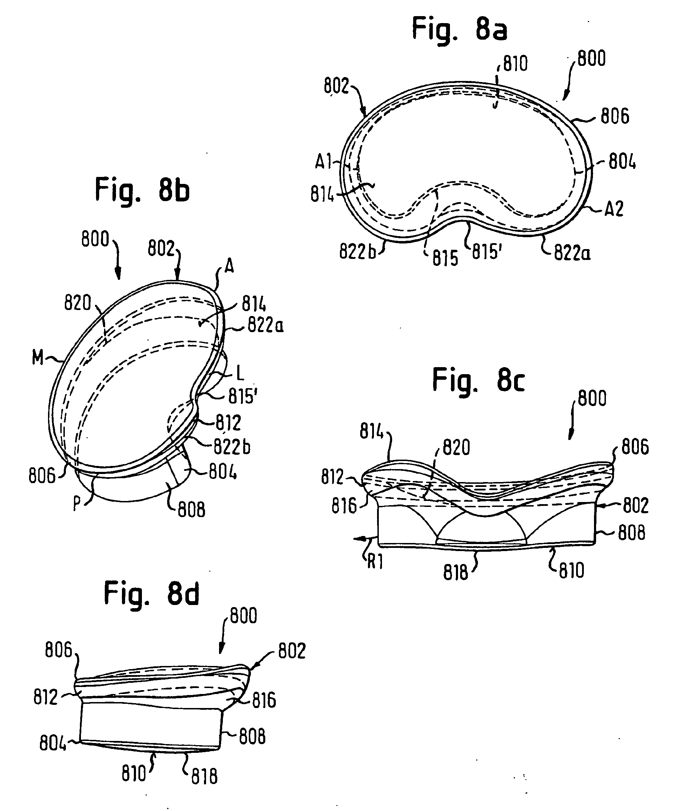

In some patients, it may be beneficial to provide an insert which includes an enlarged femoral surface. For example, in one of the examples of Figs. 8a-8d, the implantable knee prosthesis 800 comprises a body 802 having a generally elliptical shape in plan including a first portion 804 and a second portion 806. The first portion 804 has a first peripheral edge 808 and a first face 810. The second portion 806 has a second peripheral edge 812 and a second face 814. The second peripheral edge 812 extends radially outwardly, as indicated by a directional arrow designated R1, from the first peripheral edge 808 to form a shoulder 816 between the first portion 804 and the second portion 806.

The first face 810 is substantially kidney shaped in plan and the second face 814 is also substantially kidney shaped in plan. Thus, the first edge 808 and second edge 812 each preferably include an aligned notch 815, 815', respectively. The first face 810 includes a convex surface 818 and the second face 814 includes a concave surface 820. The first face 810 includes a contour which is substantially the same as an associated contour of a tibial plateau. The second face 814 includes a contour which is substantially the same as an associated contour of a femoral condyle. In this manner, the first face 810 and the second face 814 are contoured such that the prosthesis 800 is self-centering between a tibial plateau and a femoral condyle as discussed above.

The second peripheral edge 812 extends between the shoulder 816 and the second face 814 and includes a first or medial side M, a second or lateral side L, opposite the first side M, a first or anterior end A and a second or posterior end P opposite the first end A.

The second or lateral side L includes a pair of lobes 822a and 822b. The lobe 822a is adjacent the anterior end A of body 802 and the lobe 822b is adjacent the posterior end P of body 802. The notch 815' is formed between the lobes 822a, 822b so that the lobes 822a, 822b are spaced apart by the notch 815'.

The first portion 804 includes a first area A1, and the second portion 806 includes a second area A2. The extension of the second peripheral edge 812 provides for the first area A1 to be less than the second area A2.

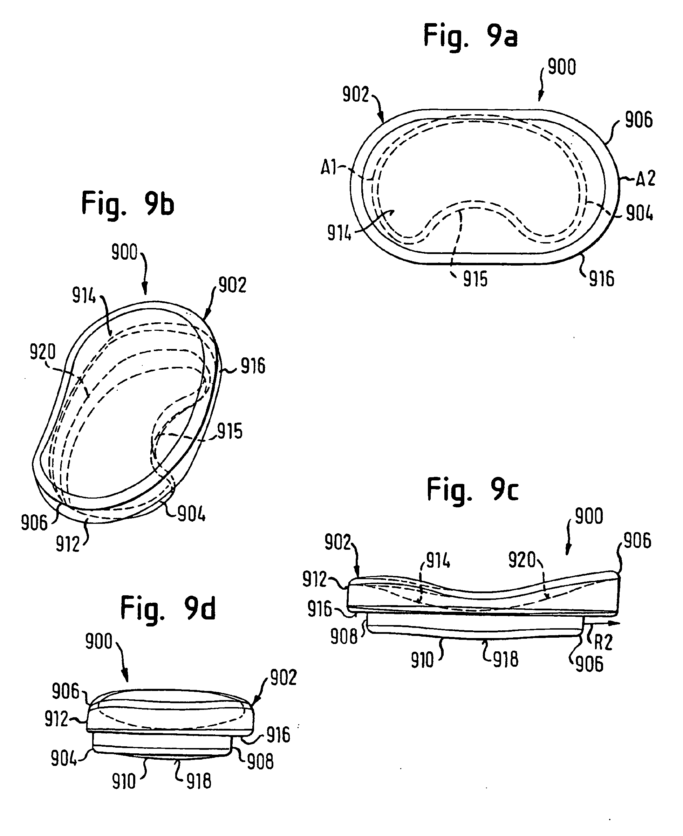

In the examples of Figs. 9a-9d, an implantable knee prosthesis 900 comprises a body 902 having a generally elliptical shape in plan including a first portion 904 and a second portion 906. The first portion 904 has a first peripheral edge 908 and a first face 910. The second portion 906 has a second peripheral edge 912 and a second face 914. The second peripheral edge 912 extends radially outwardly, as indicated by a directional arrow designated R2 from the first peripheral edge 908 to form a shoulder 916 between the first portion 904 and the second portion 906.

The first face 910 is substantially kidney shaped in plan. First edge 908 preferably includes a notch 915. The second face 914 is substantially elliptical in plan so that the shoulder 916 overhangs the notch 915. The first face 910 includes a convex surface 918 and the second face 914 includes a concave surface 920. The first face 910 includes a contour which is substantially the same as an associated contour of a tibial plateau. The second face 914 includes a contour which is substantially the same as an associated contour of a femoral condyle. In this manner, the first face 910 and the second face 914 are contoured such that the prosthesis 900 is self-centering between a tibial plateau and a femoral condyle as discussed above.

The first portion 904 includes a first area A1, and the second portion 906 includes a second area A2. The extension of the second peripheral edge 912 provides for the first area A1 to be less than the second area A2.

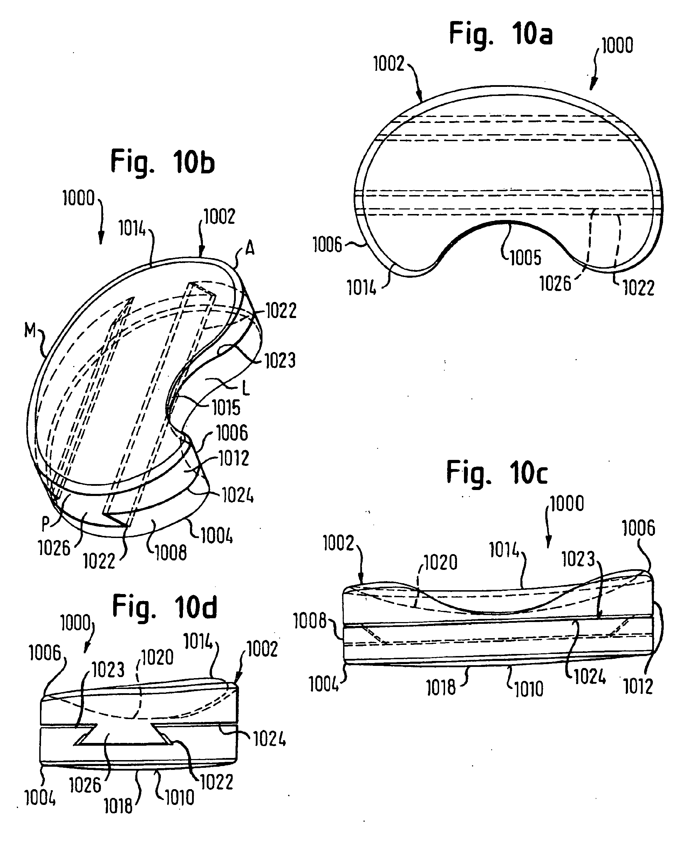

For example, in one of the examples of Figures 10a-10d, an implantable knee prosthesis 1000 comprises a two-piece body 1002 having a generally elliptical shape in plan including a first piece 1004 and a second piece 1006. The first piece 1004 has a first peripheral edge 1008 and a first face 1010. The second piece 1006 has a second peripheral edge 1012 and a second face 1014. The second peripheral edge 1012 is substantially aligned with the first peripheral edge 1008.

The first face 1010 is substantially kidney shaped in plan and the second face 1014 is also substantially kidney shaped in plan. The first peripheral edge 1008 and the second peripheral edge 1012 each preferably include an aligned notch 1015. The first face 1010 includes a convex surface 1018 and the second face 1014 includes a concave surface 1020. The first face 1010 includes a contour angle (discussed above) which is substantially the same as an associated contour angle of a tibial plateau. The second face 1014 includes a contour angle which is substantially the same as an associated contour angle of a femoral condyle. In this manner, the first face 1010 and the second face 1014 are contoured such that the prosthesis 1000 is self-centering between a tibial plateau and a femoral condyle as discussed above.

The first piece 1004 is a tibial piece and includes a first flat interface 1023 having a linear dovetail shaped keyway 1022 formed therein. The second piece 1006 is a femoral piece and includes a second flat interface 1024 for abutting with first interface 1023 and having a linear dovetail shaped key 1026 protruding therefrom, and sized for sliding engagement within keyway 1022. In this manner, the first piece 1004 and second piece 1006 are mutually slidably engagable and separable. The linear key 1026 and the mating linear keyway 1022 permit the first and second pieces 1004, 1006, respectively, to be joined in a straight-line sliding motion.

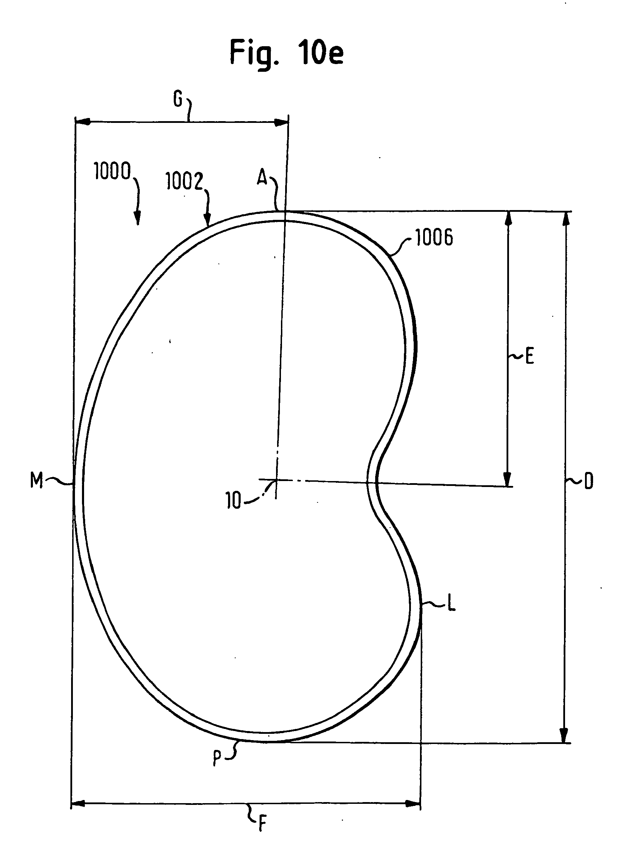

Second piece 1006, of prosthesis 1000 as viewed in Fig. 10e, is similar to prosthesis 100, Fig. 1, in that a dimension D in a range of from about .6RA to about 1.4RA, is defined by a first or anterior end A and a second or posterior end P. Another dimension F is defined by a first or medial side M and a second or lateral side L. It has been found that a suitable size for body 1002 is defined by the dimension F being from about .25RA to about 1.5A of the dimension D. Also a dimension E extends from the CSO 10 of the body 1002 to the first end A. It has also been found that a suitable size for body 1002 is also defined by the dimension E being from about .1RA to about .75RA of the dimension D. Further, a dimension G extends substantially from the CSO 10 of body 1002 to the first side M. It has further been found that a suitable size for body 1002 is further defined by the dimension G being from about .096RA to about .48RA of the dimension F.

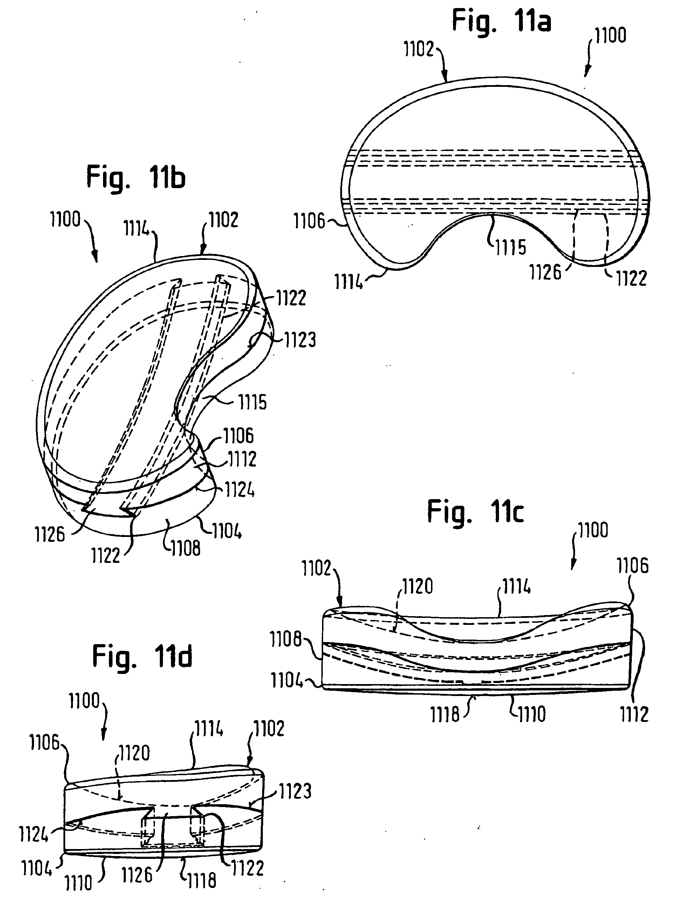

In the examples of Fig. 11a-11d, an implantable knee prosthesis 1100 comprises a two-piece body 1102 having a generally elliptical shape in plan including a first piece 1104 and a second piece 1106. The first piece 1104 has a first peripheral edge 1108 and a first face 1110. The second piece 1106 has a second peripheral edge 1112 and a second face 1114. The second peripheral edge 1112 is substantially aligned with the first peripheral edge 1108.

The first face 1110 is substantially kidney shaped in plan and the second face 1114 is also substantially kidney shaped in plan. The first peripheral edge 1108 and the second peripheral edge 1112 each preferably include an aligned notch 1115. The first face 1110 includes a convex surface 1118 and the second face 1114 includes a concave surface 1120. The first face 1110 includes a contour angle (discussed above) which is substantially the same as an associated contour angle of a tibial plateau. The second face 1114 includes a contour angle which is substantially the same as an associated contour angle of a femoral condyle. In this manner, the first face 1110 and the second face 1114 are contoured such that the prosthesis 1100 is self centering between a tibial plateau and a femoral condyle as discussed above.

The first piece 1104 is a tibial piece and includes a first concave interface 1123 having a concave dovetail shaped keyway 1122 formed therein. The second piece is a femoral piece and includes a second convex interface 1124 for abutting with first interface 1123 and having a convex dovetail shaped key 1126 protruding therefrom, and sized for sliding engagement within keyway 1122. In this manner, the first piece 1104 and second piece 1106 are mutually slidably engagable and separable. The concave keyway 1122 and, the convex key 1126 permit the first and second pieces 1104, 1106, respectively, to be joined in an arcuate scooping motion, i.e. the key 1126 may be rotated into the keyway 1122 in an arcuate motion.

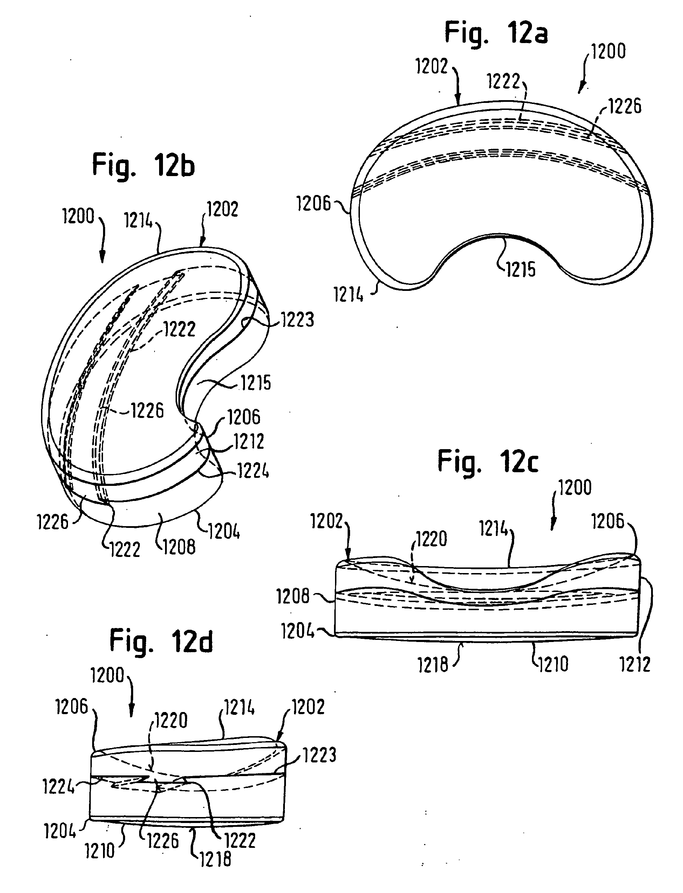

In the examples of Figures 12a-12d, an implantable knee prosthesis 1200 comprises a two-piece body 1202 having a generally elliptical shape in plan including a first piece 1204 and a second piece 1206. The first piece 1204 has a first peripheral edge 1208 and a first face 1210. The second piece 1206 has a second peripheral edge 1212 and a second face 1214. The second peripheral edge 1212 is substantially aligned with the first peripheral edge 1208.

The first face 1210 is substantially kidney shaped in plan and the second face 1214 is also substantially kidney shaped in plan. The first peripheral edge 1208 and the second peripheral edge 1212 each preferably include an aligned notch 1215. The first face 1210 includes a convex surface 1218 and the second face 1214 includes a concave surface 1220. The first face 1210 includes a contour angle (discussed above) which is substantially the same as an associated contour angle of a tibial plateau. The second face 1214 includes a contour angle which is substantially the same as an associated contour angle of a femoral condyle. In this manner, the first face 1210 and the second face 1214 are contoured such that the prosthesis 1200 is self centering between a tibial plateau and a femoral condyle as discussed above.

The first piece 1204 is a tibial piece and includes a first flat interface 1223 having a curved, or arcuate dovetail shaped keyway 1222 formed therein. The second piece 1206 is a femoral piece and includes a second flat interface 1224 for abutting with first interface 1223 and having a curved or arcuate dovetail shaped key 1226 protruding therefrom, and sized for sliding engagement within keyway 1222. In this manner, the first piece 1204 and second piece 1206 are mutually slidably engagable and separable. The curve of the key 1226 and the mating curve of the keyway 1222 permit the first and second pieces 1204, 1206, respectively, to be joined in an arcuate sweeping motion, i.e. the key 1226 may be spun or rotated into the keyway 1222 in an arcuate motion.

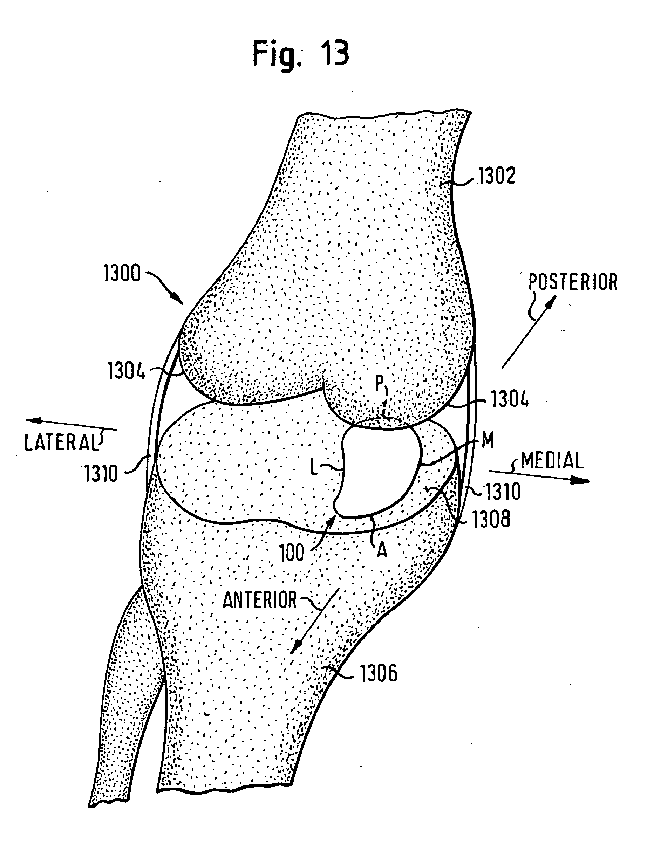

An exemplary use of, for example, prosthesis 100 is illustrated in Fig. 13. Prosthesis 100 is positioned in a knee joint 1300 between a femur 1302, including the femoral condyles 1304, and a tibia 1306 including the tibial plateau 1308. The femur 1302 and tibia 1306 include interconnecting collateral ligaments 1310. The device 100 illustrates the position of the posterior end P, the anterior end A, the medial side M and the lateral side L when the device 100 is inserted in the knee joint 1300.

The prosthetic device of the subject invention is a unicompartmental device suitable for minimally invasive, surgical implantation without requiring bone resection. The device is positioned within a compartment in which a portion of the natural meniscus is ordinarily located. The natural meniscus may be maintained in position or may be wholly or partially removed, depending upon its condition. Under ordinary circumstances, pieces of the natural meniscus which have been torn away are removed, and damaged areas may be trimmed as necessary. In somewhat rarer instances, the entire portion of the meniscus residing in the meniscal cavity may be removed. Actually, as described hereinafter, the shape of the present device is not the same as the natural meniscus, and in most cases, will not entirely replace the meniscus.

By the term "unicompartmental" is meant that each device is suitable for implantation into but one compartment defined by the space between a femoral condyle and its associated tibial plateau. In other words, the present device is not a "bicompartmental" device which, in one rigid device, could be inserted into both of the femoral condyle/tibial plateau compartments. In many, if not most cases, a device will be inserted into one compartment only, generally the medial compartment, as the meniscus and associated articular surfaces in these compartments (left knee medial and right knee medial compartments) are most subject to wear and damage. However, it is possible to insert two separate devices into the medial and lateral compartments of the same knee, or to use two such devices that are mechanically but non-rigidly linked.

The present device is translatable but self-centering. By "translatable" is meant that during natural articulation of the knee joint, the device is allowed to move, or change its position. Thus, the present device is devoid of means of physical attachment which limit its movement (for example, screws, mating ridges and depressions, porous areas to accommodate tissue regrowth, and the like).

The term "self-centering" means that upon translation from a first position to a second position during knee articulation, the device will return to substantially its original position as the articulation of the knee joint is reversed and the original knee position is reached. Thus, the device will not progressively "creep" toward one side of the compartment in which it is located. Rather, the angle of attack of the femoral condyle and/or tibial plateau bearing surfaces against the device will ensure that the device reversibly translates during articulation, maintaining the device, on average, in the same location for any given degree of knee articulation. The centered, rest, position of the implant is usually determined when the knee is in extension and there is maximum contact between the femoral condyle and the device. This ability of the device to "self-center" can be compromised by inadequate tension of either one or both of the cruciate ligaments. Unbalanced or excessive cruciate ligament tension can possibly cause the device to locate itself in a more anterior position on the tibia, which is less desirable.

Contrary to most devices which are composed of soft, compliant material designed to assume the function of the natural meniscus which they replace, the present device is composed of relatively hard, relatively high modulus material. Suitable materials are, for example, steel, ceramics, and reinforced and nonreinforced thermoset or thermoplastic polymers. The device need not be made of a single material, but composite structures of steel/thermoplastic, steel/ceramic, ceramic/polymer, etc., may be used. Alternatively, composites of the above materials with biologically active surfaces or components may be used. Biologically active components include surfaces that may contain pharmaceutical agents to stimulate cartilage growth or retard cartilage degeneration that may be delivered at once or in a timed-release manner.

Generally, portions of the device expected to have the most wear due to either greater movement relative to the mating surface (i.e., relative to the femoral condyle or tibial plateau) or high stress, may be made of stronger, more abrasion resistant material than the remainder of the device when composite structures are used. This method may be ideal for use in conjunction with cultured chondrocyte implantation (cartilage cells used as seeds) or osteochondral transplantation. Moreover, when the locus of damage to the articular cartilage or to a portion of the bone structure are known, the relatively constant radius of the surface of the present device will bridge the defective areas at these loci, thus redistributing load to healthy tissue and allowing inflamed, diseased, or other damaged areas to regenerate.

For example, a portion of the femoral condyle, tibial plateau, articular cartilage, etc., may have been damaged or may experience tissue degeneration. The continued load experienced at such points and the wear experienced as the knee flexes will substantially hinder the regeneration of healthy tissue. If suitable biologically active materials, chondrocytes, etc. are applied to the damaged or degenerated surface to assist in tissue regeneration, these will, under ordinary circumstances, be rapidly dissipated. If a flexible, cushiony material is inserted within the knee compartment, the damaged area will still experience intimate contact with the damaged area under static loads, and will also experience continued wear and abrasion under non-static conditions. Under such circumstances, active substances will be rapidly dissipated. However, more importantly, newly regenerated articular cartilage not having the necessary density or cohesiveness to withstand wear, will be rapidly eroded away.

The present device may be supplied with a contoured surface which distributes the loads evenly over regions of healthy articular cartilage while bridging areas where articular cartilage degeneration or damage has occurred. Active substances may be applied at once or in a timed-release manner to the degenerated or damaged articular cartilage surface by means of, or in conjunction with, the present device. Because the recess or shape of the device protects the damaged area from loads and wear, tissue regeneration may occur without disturbance. The regenerating tissue will have time to mature and crossline into a fully developed matrix. Moreover, as regeneration proceeds, the regenerating tissue will assume a shape dictated by the shape of the device. Growth under these circumstances has the greatest potential for dense, ordered cartilage most closely replicating the original surface.

The hardness of the present device is preferably higher than Shore 60 D. The Shore hardness may range from that common for engineering grade plastics to hardened steel and titanium, and preferably on the portion of the Rockwell hardness scale typical of steels, hard plastics and ceramic materials. From the high hardness desired of the device, it is readily apparent that the device functions in a manner completely different from those of the prior art. The purpose of the device of the subject invention is to achieve a span-like effect to bridge the defective areas. However, in a composite variation, any single component (like a bioactive material component) may be softer than the supporting material. Rather than deforming to distribute a load relatively equally on the mating surfaces, the device of the present invention functions as a rigid, substantially non-deforming, self-centering bearing, which does not necessarily spread the load uniformly, but rather may concentrate the load upon desired points, spanning areas of imperfection. If a soft and/or low modulus elastomer or thermoplastic is used for the entire device, not only is the load not concentrated on healthy tissue, but damaged areas will also be subjected to loading, thereby decreasing the opportunity for the body's natural regenerative capability to function.

The high modulus of the present device thus allows for the provision of recessed or non-contacting areas of the device to encourage articular cartilage regeneration. In softer, lower modulus materials, the naturally occurring loads, which may exceed 1000 lbs/in², in certain cases, will cause the softer devices to deform and allow ordinarily non-contracting areas to contact bone or cartilage for which contact is not desired. A flexural modulus of elasticity for load bearing portions of the present device should therefore be preferably greater than 2x10⁵ psi, and more preferably greater than 3x10⁶ psi. Portions of the device not exposed to the highest loads may be made of lower modulus materials, which may be softer as well (e.g., in a non-limiting sense, nylon, polyurethane, polypropylene, polyester, and the like, optionally fiber reinforced).

As indicated previously, the device of the subject invention may be manufactured so as to substantially contain or have deposited thereon, a biologically or pharmaceutically active material. This is particularly suitable when the device bridges a defective area of bone or articular cartilage. In such cases, the device may be provided with a coating containing a biologically or pharmaceutically active material, for example one that promotes tissue regrowth or one that decreases inflammation. Such materials may also, and more preferably, be contained in a portion of the meniscal device. The portion may be filled with medication, or may be filled with a gel, paste, or soft polymer material that releases medication over a period of time. Preferably, this medically active portion does not actually contact, or minimally contacts, the damaged tissue. This freedom from contact is made possible by the surrounding bearing surfaces. Coatings may also be of a gel, paste, or polymer containing time-release medicaments. Biologically and pharmaceutically active materials are identified subsequently herein as "active materials."

The edges of the device are rounded rather than presenting the sharp corners of the devices of U.S. Patent 5,158,574. This rounded periphery is necessary due to the fact that the device will be allowed to move within the cavity. Movement of a device having a periphery with sharp corners would result in the potential for severe damage to the surrounding tissue and articular surfaces, in addition to causing pain. A "depression" in the elliptical shape on the part of the device which will be proximate to the tibial spine will vary from patient to patient. It is possible due to the great range of variability of human anatomy that this depression might be absent in devices for some patients. However, the overall shape in plan is substantially elliptical regardless.

The axis of rotation of the tibia on the femur is 90 degrees to the path of the tibial plateau against the femoral condyle. The two tibial plateaus (medial and lateral) are not in the same plane with each other but do act in a relatively constant radius to its respective femoral condyle. In other words, although the symmetry of the device's femoral side may be matched with the femoral condyle while the leg is in full extension, the rotation of the tibial plateau against the femoral condyle is along a constant axis of rotation (90 degrees to the axis of rotation), thus the angularity of the axis of symmetry of the femoral condyle relative to the axis of symmetry of the tibial plateau is not parallel but at some acute angle. Also, the axis of symmetry of the tibial plateau is not parallel to the path of rotation of the tibia relative to the femur but also at some mildly acute angle. Thus, the true orientation of the device, regardless of the relative orientations of symmetry of the tibial side to the femoral side is 90 degrees to the true axis of rotation as described in Hollister et al., "The Axes of Rotation of the Knee", Clin. Orthopaedics and Rel. Res., 290 pp. 259-268, J.B. Lippincott Co., 1993. Any localized positions of higher loads are self-limiting due to the ability of the device to translate both rotationally and laterally which mimics the true motion of the natural meniscus as described by Hollister.

During the load bearing portion of the gait cycle, or stance phase, flexion at the knee does not typically exceed 35°. Thus, the highest compressive loads in the knee occur with the knee substantially extended. The outer contours of the device are therefore designed to substantially mate with the corresponding tibial and femoral surfaces when the knee is in full extension so that the high compressive loads can be distributed over large surface areas. The contact areas between the femoral condyle and the femoral surface of the device, and the tibial plateau and the tibial surface of the device are substantially equivalent during extension. However, because the contour of the femoral surface is more concave, the femoral condyle determines the position of the device on the surface of the tibial plateau in extension.

As the knee is flexed, the mating along the tibial surface is substantially maintained. However, the contoured mating surfaces of the femoral condyle and femoral surfaces of the present device can become increasingly dissimilar when the joint articulates. As the knee is flexed, there is a relative external rotation and posterior translation of the femur with respect to the tibia. Thus, the contour angle of the femur becomes more in-line with the contour angle of the tibia in flexion. This can cause relative lateral or rotational movement, in the tibial plane, between the femoral condyle and the femoral surface of the device. The forces generated by the increasingly different geometry creates a rotational moment, in the tibial plane, which is resisted along the mating tibial surfaces and which also results in a restoring force tending to correctly locate the device along the femoral condyle. Thus, the device is self-centering to the femoral condyle, in part, as a result of the conformity between the femoral condyle and the femoral surface of the device.

By changing the femoral surface of the implant, it is possible to reduce tile rotational moment induced during flexion by the mismatch between the femoral surface of the implant and the femoral condyle. A preferred method to accommodate this motion is to have a less acute alignment between the femoral and tibial axes of symmetry posterior to the A/P midline, thereby reducing the mismatch between the two axes in flexion. This angle is preferably 0° and can range from +/- 10°. Anterior to the midline, the femoral contour is bent around a radius RC that is tangent to the posterior section of the sweep plane at the most distal point of the femoral A/P radius RA. This femoral surface geometry is essentially a compromise between the different extension and flexion alignments of the femoral and tibial axes of symmetry.

Because the device has no physical method of attachment, the combination of the slightly concave tibial surface and the convex femoral surface serves to locate the device though all ranges of motion provided that the collateral ligaments are in proper tension. If too thin, a device could be ejected from the knee compartment. By the very nature of the ability to adjust for the lost articular material through the thickness of the device, the thickness adjustment substantially eliminates the need for a functional meniscus as a bearing surface in a severely (Grade III or IV) degenerated knee. In these instances, the femoral surface of the device resides significantly above the meniscal edge, and the meniscus is completely unloaded.

The device also increases the translational stability of the knee. The conforming shape of the femoral surface limits excessive anterior to posterior translation of the femur. As a result, this device possibly eliminates the need for ACL reconstruction in the older patient.

Generally speaking, each knee presents a different geometry of the respective femoral condyles and tibial plateaus. Even with respect to the right and left knees of a single individual, although bilateral symmetry dictates that the left and right knee components should be mirror images, this is often only an approximation. Thus, the shape of the affected femoral condyle and tibial plateau (while discussed herein in the singular, more than one pair of condyle(s)/plateau(s) may be involved), will have to be ascertained to determine the correct geometry of the device for a given patient.

To implant a device that possesses the characteristics required by the subject invention, the patient's knee joint may be examined by a non-invasive imaging procedure capable of generating sufficient information such that one appropriately sized and shaped device may be selected. While a variety of non-invasive imaging devices may be suitable, for example X-ray devices and the like, it is preferable that information as to the size and shape of the device be provided by magnetic resonance imaging (MRI).

Two methods of non-invasive imaging for selection of a suitable prosthesis are preferred. In the first method, MRI or other non-invasive imaging scans, optionally coupled with exterior measurements of the dimensions of the relevant tibial and femoral portions including the surface of the particular cartilage of the tibia and femur, may be used to establish a library of prostheses whose size and geometry differ according to the age and size of the patient, the patient's genetic make-up, and the like. A limited number of "standard" devices are then made to meet the requirements of a generic population of patients.

In this first method, a non-invasive imaging scan, such as X-ray or MRI, together with knowledge of the patient's genetic make-up, general body type, extent of the disease, degeneration, or trauma and the like, will enable the surgeon to select a device of the correct size and shape from the library for the patient. The device is then introduced by arthroscopically assisted implantation, generally limited to extensive clean-up of existing damaged tissue, e.g., torn or particulate natural meniscus damage. It may also be used in conjunction with tibial osteotomy or articular surfacing procedure such as cartilage transplantations or abrasion anthroplasty. Following insertion of the device, X-ray, Fluoroscopy, or MRI may be used to assess the correct positioning of the device both introperatively as well as postoperatively. Because the surgical procedures used are not severe, and also not irreversible, an unsuitable device may be readily removed and replaced, either with a different device from a device library, or by a custom device.

In a second method, each patient receives one or more devices that are custom tailored for the individual by producing a contour plot of the femoral and tibial mating surfaces and the size of the meniscal cavity. Such a contour plot may be constructed from imaging data, i.e. MRI data, by a suitable computer program. From the contour plot, the correct surface geometry of the device is determined from the shape of the respective tibial plateau and femoral condyle and the orientation between the two surfaces in extension. In general, the shapes just mentioned also include the articular cartilage, which, in general, is maintained substantially intact.

It has been discovered that the amount of varus deformity is the primary, non-invasive method for determining the necessary device thickness required for proper functioning of the device. Viewing a weight bearing anteroposterior X-ray, a cut and paste of a line drawn through the femoral condyles and repositioned to put them once again parallel to the tibial plateaus will yield a measurement for the approximate device thickness.

A further understanding can be obtained by reference to the following specific example that is provided herein for purposes of illustration only and is not intended to be limiting unless otherwise specified.

### For Example:

A 44-year-old male had an 18-degree flexion contracture in his right knee. The right limb was in 5 degrees of varus alignment and the patient suffered from significant, debilitating pain. X-rays of the affected limb showed significant collapse of the medial joint space as well as significant osteophyte formation along the medial border of the femoral condyle. Pre-operative templating of the X-ray indicated that the right medial condyle had a radius of approximately 46mm. A library of implants was manufactured for this patient based upon the preoperative radius measurement and the dimensional relationships established from the X-ray and MRI database. The library included implants with a femoral radius measuring 42mm, 46mm and 50mm. Implants of 2mm, 3mm and 4mm thickness were made in each size category. The patient was then scheduled for surgery.

Arthroscopic evaluation of the joint on the day of surgery revealed generalized Grade III chondromalicia of the medial femoral condyle and tibial plateau with small areas of Grade IV changes. Patellofemoral and lateral joint compartment changes were mild. An arthroscopic debridement of the joint was completed and the degenerated edge of the meniscus was resected. A small ruler was inserted through the anterior arthroscopic portal and the distance from the posterior rim to the anterior rim of the remaining meniscus was recorded as 42mm. A short median parapatellar incision was completed to expose the medial compartment of the knee. An osteotome and a rongure were used to remove the osteophytes along the medial border of the femoral condyle. Plastic gages representing the different implant thicknesses were then inserted between the femur and the tibia to measure the amount of joint space present in the medial compartment. These measurements indicated that a 4mm thick part would be required to occupy the joint space and restore tension to the medial compartment. Several trial implants were inserted into the joint space and a fluoroscope was used to verify the fit and positioning of each trial. This final trial reduction confirmed that the appropriate part was a 42mm long by 4mm thick implant. The implant was inserted into the joint. A final check of the implant's stability and fit was performed. Careful attention was paid to the evaluation of implant thickness because an inappropriately thick implant could prevent the patient from achieving full extension. After all interoperative checks were complete the incision was closed.

Postoperative X-rays revealed a 7-degree correction of the limb alignment. The implant also stabilized the knee. At 10 months of follow-up the patient is pain free and can achieve full knee extension. The patient can also achieve approximately 120 degrees of flexion.

One preferred surgical procedure which may be used to implant this device can be described by the following steps:
1. Verify preoperative indications:
   a. Varus determination of < 5 degrees with erect AP X-ray;
   b. Medial compartment disease only. Some lateral spurs may be present; and
   c. Pre-operative sizing via M/L template measurement of A/P X-ray.
2. Standard Arthroscopy surgical prep:
   a. Infiltrate knee with Lidocaine/Marcaine and Epinephine.
3. Arthroscopy:
   a. Inspect lateral patello-femoral compartments for integrity, some mild arthrosis is acceptable;
   b. Removal of medial meniscus toward the rim along the anterior, medial and posterior portions;
   c. Initial arthroscopic osteophyte removal via 1/8" osteotome and burr to allow for valgus positioning of the knee;
   d. Complete the removal (to the rim) of the posterior and posterior-lateral meniscus; and
   e. Confirm sizing of the device by measuring distance from resected posterior meniscus to remaining anterior meniscus.
4. Medial parapatellar arthrotomy (mid-patella to tibial joint line).
5. Complete removal of visible osteophytes along the medial femoral condyle.
6. Insert thickness gauge and size for implant thickness.
7. Insert trial component:
   a. Flex knee to approximately 50+ degrees to fully expose the distal portion of the femoral condyle;
   b. Insert trial component; and
   c. While applying insertion pressure, apply valgus stress to the tibia and "stretch-extend" the tibia over the trial component.
8. Check for proper sizing with "true lateral" and A/P fluoroscope images of the knee while in extension:
   a. Ideally, the device should be within 1mm of the A/P boundries of the tibial plateau and superimposed over the medial boundary.
9. Remove trial component and flush joint with saline.
10. Insert the appropriate implant.
11. Confirm proper placement and sizing with fluoroscopic images as with trial component.
12. Maintain leg in extension and close wound after insertion of a Hemovac drain.
13. Place leg in immobilizer prior to patient transfer.

## Claims

1. Implantable knee prosthesis comprising:
a body (402; 502) having a shape in plan with rounded corners that is longer in a first dimension than a second dimension transverse to the first dimension, a tibial face (404; 504), and an opposing femoral face (408; 508);
a peripheral edge (412; 512) of variable thickness extending between the faces (504; 508) and having a first side (M), a second side (L) opposite the first side (M), a first end (A) and a second end (P) opposite the first end (A); and
the thickness of the peripheral edge (412; 512) at the first side (M) being greater than the thickness of the peripheral edge (412; 512) at the second side (L), the first end (A) and the second end (P),
**characterized in that**
the tibial face (404; 504) includes a first portion (A) and a second portion (B), the second portion extending at an angle (a) relative to the first portion.

2. Knee prosthesis in accordance with claim 1,
**characterized in that**
one of the faces includes a first portion and a second portion, the second portion extending at an angle with the first portion.

3. Knee prosthesis in accordance with claim 1 or claim 2,
**characterized in that**
the body is a one-piece body.

4. Knee prosthesis in accordance with claim 1 or claim 2,
**characterized in that**
the body includes a first piece, attached to a second piece.

5. Knee prosthesis in accordance with claim 4,
**characterized in that**
the first piece and the second piece are mutually slidably engagable and separable.

6. Knee prosthesis in accordance with claim 5,
**characterized in that**
the first and second pieces are keyed for sliding engagement.

7. Knee prosthesis in accordance with any one of the claims 4 to 6,
**characterized in that**
the first piece includes a first face and the second piece includes a second face, the second face being at an angle with the first face.

8. Knee prosthesis in accordance with any one of the preceding claims,
**characterized in that**
a first dimension D is defined by the first end and the second end, and a second dimension F defined by the first side and the second side, where the dimension F is from about .25 to about 1.5 of the dimension D.

9. Knee prosthesis in accordance with claim 8,
**characterized in that**
one of the faces is a femoral face including an anterior to posterior radius RA and the first dimension D, where D is from about .6RA to about 1.4RA.

10. Knee prosthesis in accordance with claim 9,
**characterized in that**
the anterior to posterior radius RA is equal to the first dimension D.

11. Knee prosthesis in accordance with any of the claims 8 to 10,
**characterized in that**
the second dimension F is R64A.

12. Knee prosthesis in accordance with any of the claims 9 to 11,
**characterized in that**
the femoral face includes a medial to lateral radius B of from about 25RA to about 1.0RA.

13. Knee prosthesis in accordance with claim 12,
**characterized in that**
the radius B is 68RA.

14. Knee prosthesis in accordance with any of the claims 9 to 13,
**characterized in that**
the femoral face includes an anterior sweep radius RC to accommodate relative rotation between femur and a tibia.

15. Knee prosthesis in accordance with claim 14,
**characterized in that**
RC is from about 5RA to about 2.0RA.

16. Knee prosthesis in accordance with claim 15,
**characterized in that**
RC = RA.

17. Knee prosthesis in accordance with any one of the preceding claims,
**characterized in that**
a first one (604; 704) of the faces (604, 606; 704, 706) has a first shape;
a second one (606; 706) of the faces (604, 606; 704, 706) has a second shape different from the first shape; and
the peripheral edge (612; 712) extending at an incline between the first face (604; 704) and the second face (606; 706).

18. Knee prosthesis in accordance with claim 17,
**characterized in that**
the first face is substantially kidney shaped in plan.

19. Knee prosthesis in accordance with any one of the claims 17 to 18,
**characterized in that**
the first face includes a convex surface.

20. Knee prosthesis in accordance with any one of the claims 17 to 19,
**characterized in that**
the second face includes a concave surface.

21. Knee prosthesis in accordance with any one of the claims 17 to 20,
**characterized in that**
the first face includes a contour which is substantially the same as an associated contour of a tibial plateau.

22. Knee prosthesis in accordance with any one of the claims 17 to 21,
**characterized in that**
the second face includes a contour which is substantially the same as an associated contour of a femoral condyle.

23. Knee prosthesis in accordance with any one of the claims 17 to 22,
**characterized in that**
the peripheral edge adjacent the first face includes a notch formed therein.

24. Knee prosthesis in accordance with claim 23,
**characterized in that**
the second face extends over the notch.

25. Knee prosthesis in accordance with any one of the claims 17 to 24,
**characterized in that**
the second face is offset relative to the first face.

26. Knee prosthesis in accordance with any one of the claims 17 to 25,
**characterized in that**
the first face has a first area and the second face has a second area different than the first area.

27. Knee prosthesis in accordance with any one of the claims 17 to 26,
**characterized in that**
the peripheral edge adjacent the first one of the faces includes a first side, a second side opposite the first side, a first end, a second end opposite the first end, a first dimension D defined by the first end and the second end, and a second dimension F defined by the first side and the second side, where the dimension F is from about .25 to about 1.5 of the dimension D.

28. Knee prosthesis in accordance with any one of the preceding claims,
**characterized in that**
the body (802; 902) includes a first portion (804; 904) and second portion (806; 906);
the first portion (804; 904) having a first peripheral edge (808; 908) and a first face (810; 910);
the second portion (806; 906) having a second peripheral edge (812; 912) and a second face (814; 914); and
the second peripheral edge (812; 912) extending radially outwardly from the first peripheral edge (808; 908) to form a shoulder (816; 916) between the first portion (804; 904) and the second portion (806; 906).

29. Knee prosthesis in accordance with claim 28,
**characterized in that**
the first peripheral edge includes a first side, a second side opposite the first side, a first end, a second end opposite the first end, a first dimension D defined by the first end and the second end, and a second dimension F defined by the first side and the second side, where the dimension F is from about .25 to about 1.5 of the dimension D.

30. Knee prosthesis in accordance with any one of the claims 19, 20 and/or 28 to 29,
**characterized in that**
the first portion is substantially kidney shaped including a first notch.

31. Knee prosthesis in accordance with any one of the claims 19, 20 and/or 28 to 30,
**characterized in that**
the second portion is substantially kidney shaped including a second notch.

32. Knee prosthesis in accordance with claim 31,
**characterized in that**
the second notch extends radially outwardly from the first notch.

33. Knee prosthesis in accordance with any one of the claims 19, 20 and/or 28 to 32,
**characterized in that**
the first portion includes a first area A1, and the second portion includes a second area A2, different from the first area A1.

34. Knee prosthesis in accordance with claim 33,
**characterized in that**
the first area A1 is less than the second area A2.

35. Knee prosthesis in accordance with any one of the claims 19, 20 and/or 28 to 34,
**characterized in that**
the first peripheral edge includes a first notch and the second peripheral edge includes a second notch aligned with the first notch.

36. Knee prosthesis in accordance with any one of the claims 19, 20 and/or 28 to 34,
**characterized in that**
only the first peripheral edge includes a notch.

37. Knee prosthesis in accordance with any one of the preceding claims,
**characterized in that**
the body (1002; 1102; 1202) is a two-piece body;
a first piece (1004; 1104; 1204) being a tibial piece including a tibial surface;
a second piece (1006; 1106; 1206) being a femoral piece including a femoral surface;
the first piece (1004; 1104; 1204) and the second piece (1006; 1106; 1206) being mutually slidably engagable and separable.

38. Knee prosthesis in accordance with any one of the claims 11 to 16 or 37,
**characterized in that**
the first and second pieces are interconnected by a key engaged in a keyway.

39. Knee prosthesis in accordance with any one of the claims 11 to 16 or 38,
**characterized in that**
the key and keyway are substantially linear.

40. Knee prosthesis in accordance with any one of the claims 11 to 16 or 38,
**characterized in that**
the key and keyway are substantially arcuate.

41. Knee prosthesis in accordance with any one of the claims 11 to 16 or 37 to 40,
**characterized in that**
the first and second pieces each have an interface surface.

42. Knee prosthesis in accordance with any one of the claims 11to 16 or 41,
**characterized in that**
each interface surface is substantially flat.

43. Knee prosthesis in accordance with any one of the claims 11 to 16 or 41,
**characterized in that**
each interface surface is substantially arcuate.

44. Knee prosthesis in accordance with any one of the claims 11 to 16 or 37 to 43,
**characterized by**
a first side, a second side opposite the first side, a first end, a second end opposite the first end, a first dimension D defined by the first end and the second end, and a second dimension F defined by the first side and the second side, where the dimension F is from about .25 to about 1.5 of the dimension D.

45. Knee prosthesis in accordance with any one of the claims 10 to 16 or 37 to 44,
**characterized in that**
the femoral surface includes an anterior to posterior radius RA and the first dimension D, where D is from about 6RA to about 1.4RA.

46. Knee prosthesis in accordance with any one of the claims 12 to 16 or 45,
**characterized in that**
the anterior to posterior radius RA is equal to the first dimension D.

47. Knee prosthesis in accordance with any one of the claims 37 to 46,
**characterized by**
the first piece having a first notch and the second piece having a second notch aligned with the first notch.

## Patentansprüche

1. Implantierbare Knieprothese mit:
einem Körper (402; 502), der in Draufsicht abgerundete Ecken hat und in einer ersten Ausdehnung länger ist, als in einer zweiten Ausdehnung quer zur ersten Ausdehnung, einer Tibiafläche (404; 504) und einer gegenüberliegenden Femurfläche (408; 508);
wobei sich eine Außenkante (412; 512) mit variabler Stärke zwischen den Flächen (504; 508) erstreckt und eine erste Seite (M), eine zweite Seite (L), die der ersten Seite (M) gegenüberliegt, ein erstes Ende (A) und ein zweites Ende (P), das dem ersten Ende (A) gegenüberliegt, hat; und
wobei die Stärke der Außenkante (412; 512) an der ersten Seite (M) größer ist, als die Stärke der Außenkante (412; 512) an der zweiten Seite (L), dem ersten Ende (A) und dem zweiten Ende (P),
**dadurch gekennzeichnet, dass**
die Tibiafläche (404; 504) einen ersten Abschnitt (A) und einen zweiten Abschnitt (B) aufweist, wobei sich der zweite Abschnitt in Bezug zum ersten Abschnitt in einem Winkel (a) erstreckt.

2. Knieprothese nach Anspruch 1,
**dadurch gekennzeichnet, dass**
eine der Flächen einen ersten Abschnitt und einen zweiten Abschnitt aufweist, wobei sich der zweite Abschnitt in einem Winkel zum ersten Abschnitt erstreckt.

3. Knieprothese nach Anspruch 1 oder Anspruch 2,
**dadurch gekennzeichnet, dass**
der Körper ein einstückiger Körper ist.

4. Knieprothese nach Anspruch 1 oder Anspruch 2,
**dadurch gekennzeichnet, dass**
der Körper ein erstes Stück aufweist, das an einem zweiten Stück befestigt ist.

5. Knieprothese nach Anspruch 4,
**dadurch gekennzeichnet, dass**
das erste Stück und das zweite Stück gleitend miteinander in Eingriff gebracht und voneinander getrennt werden können.

6. Knieprothese nach Anspruch 5,
**dadurch gekennzeichnet, dass**
das erste und das zweite Stück zum gleitenden Eingriff formschlüssig sind.

7. Knieprothese nach einem der Ansprüche 4 bis 6,
**dadurch gekennzeichnet, dass**
das erste Stück eine erste Fläche aufweist und das zweite Stück eine zweite Fläche aufweist, wobei die zweite Fläche mit der ersten Fläche in einem Winkel angeordnet ist.

8. Knieprothese nach einem der vorhergehenden Ansprüche,
**dadurch gekennzeichnet, dass**
die erste Ausdehnung D durch das erste Ende und das zweite Ende definiert ist und eine zweite Ausdehnung F durch die erste Seite und die zweite Seite definiert ist, wobei die Ausdehnung F ca. 0,25 bis ca. 1,5 der Ausdehnung D beträgt.

9. Knieprothese nach Anspruch 8,
**dadurch gekennzeichnet, dass**
eine der Flächen eine Femurfläche ist, die einen Anterior-Posterior-Radius RA und die erste Ausdehnung D aufweist, wobei D ca. 6RA bis ca. 1,4RA beträgt.

10. Knieprothese nach Anspruch 9,
**dadurch gekennzeichnet, dass**
der Anterior-Posterior-Radius RA gleich der ersten Ausdehnung D ist.

11. Knieprothese nach einem der Ansprüche 8 bis 10,
**dadurch gekennzeichnet, dass**
die zweite Ausdehnung F 0,64RA ist.

12. Knieprothese nach einem der Ansprüche 9 bis 11,
**dadurch gekennzeichnet, dass**
die Femurfläche einen Medial-Lateral-Radius B von ca. 0,25RA bis ca. 1,0RA aufweist.

13. Knieprothese nach Anspruch 12,
**dadurch gekennzeichnet, dass**
der Radius B 0,68RA beträgt.

14. Knieprothese nach einem der Ansprüche 9 bis 13,
**dadurch gekennzeichnet, dass**
die Femurfläche einen anterioren Kurvenschwenkradius RC aufweist, um die relative Drehung zwischen Femur und Tibia aufzunehmen.

15. Knieprothese nach Anspruch 14,
**dadurch gekennzeichnet, dass**
RC von ca. 0,5RA bis ca. 2.0RA beträgt.

16. Knieprothese nach Anspruch 15,
**dadurch gekennzeichnet, dass**
RC = RA.

17. Knieprothese nach einem der vorhergehenden Ansprüche,
**dadurch gekennzeichnet, dass**
eine erste (604; 704) der Flächen (604, 606; 704, 706) eine erste Form hat;
eine zweite (606; 706), der Flächen (604, 606; 704, 706) eine zweite Form hat, die sich von der ersten Form unterscheidet; und
die Außenkante (612; 712) sich mit einer Neigung zwischen der ersten Fläche (604; 704) und der zweiten Fläche (606; 706) erstreckt.

18. Knieprothese nach Anspruch 17,
**dadurch gekennzeichnet, dass**
die erste Fläche in Draufsicht im Wesentlichen nierenförmig ist.

19. Knieprothese nach einem der Ansprüche 17 bis 18,
**dadurch gekennzeichnet, dass**
die erste Fläche eine konvexe Oberfläche aufweist.

20. Knieprothese nach einem der Ansprüche 17 bis 19,
**dadurch gekennzeichnet, dass**
die zweite Fläche eine konkave Oberfläche aufweist.

21. Knieprothese nach einem der Ansprüche 17 bis 20,
**dadurch gekennzeichnet, dass**
die erste Fläche eine Kontur aufweist, die im Wesentlichen gleich einer zugehörigen Kontur eines Tibiakopfs ist.

22. Knieprothese nach einem der Ansprüche 17 bis 21,
**dadurch gekennzeichnet, dass**
die zweite Fläche eine Kontur aufweist, die im Wesentlichen gleich einer zugehörigen Kontur eines Femurkondylus ist.

23. Knieprothese nach einem der Ansprüche 17 bis 22,
**dadurch gekennzeichnet, dass**
die Außenkante nahe der ersten Fläche eine darin ausgebildete Kerbe aufweist.

24. Knieprothese nach Anspruch 23,
**dadurch gekennzeichnet, dass**
sich die zweite Fläche über die Kerbe erstreckt.

25. Knieprothese nach einem der Ansprüche 17 bis 24,
**dadurch gekennzeichnet, dass**
die zweite Fläche in Bezug zur ersten Fläche versetzt ist.

26. Knieprothese nach einem der Ansprüche 17 bis 25,
**dadurch gekennzeichnet, dass**
die erste Fläche einen ersten Bereich hat und die zweite Fläche einen zweiten Bereich hat, der sich vom ersten Bereich unterscheidet.

27. Knieprothese nach einem der Ansprüche 17 bis 26,
**dadurch gekennzeichnet, dass**
die Außenkante nahe der ersten der Flächen eine erste Seite aufweist, eine zweite Seite gegenüber der ersten Seite, ein erstes Ende, ein zweites Ende gegenüber dem ersten Ende, eine erste Ausdehnung D, die durch das erste Ende und das zweite Ende definiert ist, und eine zweite Ausdehnung F, die durch die erste Seite und die zweite Seite definiert ist, wobei die Ausdehnung F von ca. 0,25 bis ca. 1,5 der Ausdehnung D beträgt.

28. Knieprothese nach einem der vorhergehenden Ansprüche,
**dadurch gekennzeichnet, dass**
der Körper (802; 902) einen ersten Abschnitt (804; 904) und einen zweiten Abschnitt (806; 906) aufweist;
wobei der erste Abschnitt (804; 904) eine erste Außenkante (808; 908) und eine erste Fläche (810; 910) hat;
wobei der zweite Abschnitt (806; 906) eine zweite Außenkante (812; 912) und eine zweite Fläche (814; 914) hat; und
wobei sich die zweite Außenkante (812; 912) von der ersten Außenkante (808; 908) radial nach außen erstreckt, um eine Schulter (816; 916) zwischen dem ersten Abschnitt (804; 904) und dem zweiten Abschnitt (806; 906) zu bilden.

29. Knieprothese nach Anspruch 28,
**dadurch gekennzeichnet, dass**
die erste Außenkante eine erste Seite aufweist, eine zweite Seite gegenüber der ersten Seite, ein erstes Ende, ein zweites Ende gegenüber dem ersten Ende, eine erste Ausdehnung D, die durch das erste Ende und das zweite Ende definiert ist, und eine zweite Ausdehnung F, die durch die erste Seite und die zweite Seite definiert ist, wobei die Ausdehnung F von ca. 0,25 bis ca. 1,5 der Ausdehnung D beträgt.

30. Knieprothese nach einem der Ansprüche 19, 20 und/oder 28 bis 29,
**dadurch gekennzeichnet, dass**
der erste Abschnitt im Wesentlichen nierenförmig ist und eine erste Kerbe aufweist.

31. Knieprothese nach einem der Ansprüche 19, 20 und/oder 28 bis 29,
**dadurch gekennzeichnet, dass**
der zweite Abschnitt im Wesentlichen nierenförmig ist und eine zweite Kerbe aufweist.

32. Knieprothese nach Anspruch 31,
**dadurch gekennzeichnet, dass**
sich die zweite Kerbe von der ersten Kerbe radial nach außen erstreckt.

33. Knieprothese nach einem der Ansprüche 19, 20 und/oder 28 bis 32,
**dadurch gekennzeichnet, dass**
der erste Abschnitt einen ersten Bereich A1 aufweist und der zweite Abschnitt einen zweiten Bereich A2 aufweist, der sich vom ersten Bereich A1 unterscheidet.

34. Knieprothese nach Anspruch 33,
**dadurch gekennzeichnet, dass**
der erste Bereich A1 kleiner ist, als der zweite Bereich A2.

35. Knieprothese nach einem der Ansprüche 19, 20 und/oder 28 bis 34,
**dadurch gekennzeichnet, dass**
die erste Außenkante eine erste Kerbe aufweist und die zweite Außenkante eine zweite Kerbe aufweist, die mit der ersten Kerbe fluchtet.

36. Knieprothese nach einem der Ansprüche 19, 20 und/oder 28 bis 34,
**dadurch gekennzeichnet, dass**
nur die erste Außenkante eine Kerbe aufweist.

37. Knieprothese nach einem der vorhergehenden Ansprüche,
**dadurch gekennzeichnet, dass**
der Körper (1002; 1102; 1202) ein zweistückiger Körper ist; wobei ein erstes Stück (1004; 1104; 1204) ein Tibiastück mit einer Tibiaoberfläche ist;
wobei ein zweites Stück (1006; 1106; 1206) ein Femurstück mit einer Femuroberfläche ist;
wobei das erste Stück (1004; 1104; 1204) und das zweite Stück (1006; 1106; 1206) gleitend miteinander in Eingriff gebracht und voneinander getrennt werden können.

38. Knieprothese nach einem der Ansprüche 11 bis 16 oder 37,
**dadurch gekennzeichnet, dass**
das erste und das zweite Stück durch einen Keil, der in eine Keilnut eingreift, miteinander verbunden sind.

39. Knieprothese nach einem der Ansprüche 11 bis 16 oder 38,
**dadurch gekennzeichnet, dass**
der Keil und die Keilnut im Wesentlichen linear sind.

40. Knieprothese nach einem der Ansprüche 11 bis 16 oder 38,
**dadurch gekennzeichnet, dass**
der Keil und die Keilnut im Wesentlichen gebogen sind.

41. Knieprothese nach einem der Ansprüche 11 bis 16 oder 37 bis 40,
**dadurch gekennzeichnet, dass**
das erste und das zweite Stück jeweils eine Grenzoberfläche haben.

42. Knieprothese nach einem der Ansprüche 11 bis 16 oder 41,
**dadurch gekennzeichnet, dass**
jede Grenzoberfläche im Wesentlichen eben ist.

43. Knieprothese nach einem der Ansprüche 11 bis 16 oder 41,
**dadurch gekennzeichnet, dass**
jede Grenzoberfläche im Wesentlichen gebogen ist.

44. Knieprothese nach einem der Ansprüche 11 bis 16 oder 37 bis 43,
**gekennzeichnet durch**
eine erste Seite, eine zweite Seite gegenüber der ersten Seite, ein erstes Ende, ein zweites Ende gegenüber dem ersten Ende, eine erste Ausdehnung D, die **durch** das erste Ende und das zweite Ende definiert ist, und eine zweite Ausdehnung F, die **durch** die erste Seite und die zweite Seite definiert ist, wobei die Ausdehnung F ca. 0,25 bis ca. 1,5 der Ausdehnung D beträgt.

45. Knieprothese nach einem der Ansprüche 10 bis 16 oder 37 bis 44,
**dadurch gekennzeichnet, dass**
die Femuroberfläche einen Anterior-Posterior-Radius RA und die erste Ausdehnung D aufweist, wobei D von ca. 0,6RA bis ca. 1,4RA beträgt.

46. Knieprothese nach einem der Ansprüche 12 bis 16 oder 45,
**dadurch gekennzeichnet, dass**
der Anterior-Posterior-Radius RA gleich der ersten Ausdehnung D ist.

47. Knieprothese nach einem der Ansprüche 37 bis 46,
**dadurch gekennzeichnet, dass**
das erste Stück eine erste Kerbe hat und das zweite Stück eine zweite Kerbe hat, die mit der ersten Kerbe fluchtet.

## Revendications

1. Prothèse de genou implantable comprenant :
un corps (402 ; 502) ayant une forme en plan avec des coins arrondis, qui est plus long dans une première dimension qu'une deuxième dimension transversale par rapport à la première dimension, une face tibiale (404 ; 504) et une face fémorale opposée (408 ; 508) ;
un bord périphérique (412 ; 512) d'épaisseur variable s'étendant entre les faces (504 ; 508) et ayant un premier côté (M), un deuxième côté (L) opposé au premier côté (M), une première extrémité (A) et une deuxième extrémité (P) opposée à la première extrémité (A) ; et
l'épaisseur du bord périphérique (412 ; 512) sur le premier côté (M) étant supérieure à l'épaisseur du bord périphérique (412 ; 512) sur le deuxième côté (L), la première extrémité (A) et la deuxième extrémité (P),
**caractérisée en ce que**
la face tibiale (404 ; 504) comprend une première partie (A) et une deuxième partie (B), la deuxième partie s'étendant à un angle (α) par rapport à la première partie.

2. Prothèse de genou selon la revendication 1, **caractérisée en ce que**
l'une des faces comprend une première partie et une deuxième partie, la deuxième partie s'étendant à un angle avec la première partie.

3. Prothèse de genou selon la revendication 1 ou la revendication 2, **caractérisée en ce que**
le corps est un corps d'un seul tenant.

4. Prothèse de genou selon la revendication 1 ou la revendication 2, **caractérisée en ce que**
le corps comprend un premier élément fixé à un deuxième élément.

5. Prothèse de genou selon la revendication 4, **caractérisée en ce que**
le premier élément et le deuxième élément peuvent coulisser de façon à pouvoir être en prise l'un avec l'autre et être séparés.

6. Prothèse de genou selon la revendication 5, **caractérisée en ce que**
les premier et deuxième éléments sont verrouillés pour la mise en prise par coulissement.

7. Prothèse de genou selon l'une quelconque des revendications 4 à 6, **caractérisée en ce que**
le premier élément comprend une première face et le deuxième élément comprend une deuxième face, la deuxième face étant à un angle avec la première face.

8. Prothèse de genou selon l'une quelconque des revendications précédentes, **caractérisée en ce que**
une première dimension D est définie par la première extrémité et la deuxième extrémité, et une deuxième dimension F est définie par le premier côté et le deuxième côté, la dimension F étant d'environ 0,25 à environ 1,5 de la dimension D.

9. Prothèse de genou selon la revendication 8, **caractérisée en ce que**
l'une des faces est une face fémorale comprenant un rayon RA antérieur à postérieur et la première dimension D, D étant d'environ 0,6 RA à environ 1,4 RA.

10. Prothèse de genou selon la revendication 9, **caractérisée en ce que**
le rayon RA antérieur à postérieur est égal à la première dimension D.

11. Prothèse de genou selon l'une quelconque des revendications 8 à 10, **caractérisée en ce que**
la deuxième dimension F est de 0,64 RA.

12. Prothèse de genou selon l'une quelconque des revendications 9 à 11, **caractérisée en ce que**
la face fémorale comprend un rayon B médial à latéral d'environ 0,25 RA à environ 1,0 RA.

13. Prothèse de genou selon la revendication 12, **caractérisée en ce que**
le rayon B est de 0,68 RA.

14. Prothèse de genou selon l'une quelconque des revendications 9 à 13, **caractérisée en ce que**
la face fémorale comprend un rayon de balayage antérieur RC pour suivre une rotation relative entre un fémur et un tibia.

15. Prothèse de genou selon la revendication 14, **caractérisée en ce que**
RC est d'environ 0,5 RA à environ 2,0 RA.

16. Prothèse de genou selon la revendication 15, **caractérisée en ce que**
RC = RA.

17. Prothèse de genou selon l'une quelconque des revendications précédentes, **caractérisée en ce que**
une première (604 ; 704) des faces (604, 606 ; 704, 706) a une première forme ;
une deuxième (606 ; 706) des faces (604, 606 ; 704, 706) a une deuxième forme différente de la première forme ; et
le bord périphérique (612 ; 712) s'étend sur un plan incliné entre la première face (604 ; 704) et la deuxième face (606 ; 706).

18. Prothèse de genou selon la revendication 17, **caractérisée en ce que**
la première face est substantiellement en forme de haricot dans le plan.

19. Prothèse de genou selon l'une quelconque des revendications 17 à 18, **caractérisée en ce que**
la première face comprend une surface convexe.

20. Prothèse de genou selon l'une quelconque des revendications 17 à 19, **caractérisée en ce que**
la deuxième face comprend une surface concave.

21. Prothèse de genou selon l'une quelconque des revendications 17 à 20, **caractérisée en ce que**
la première face comprend un contour qui est substantiellement le même qu'un contour associé d'un plateau tibial.

22. Prothèse de genou selon l'une quelconque des revendications 17 à 21, **caractérisée en ce que**
la deuxième face comprend un contour qui est substantiellement le même qu'un contour associé d'un condyle fémoral.

23. Prothèse de genou selon l'une quelconque des revendications 17 à 22, **caractérisée en ce que**
le bord périphérique adjacent à la première face comprend une encoche formée dans celui-ci.

24. Prothèse de genou selon la revendication 23, **caractérisée en ce que**
la deuxième face s'étend sur l'encoche.

25. Prothèse de genou selon l'une quelconque des revendications 17 à 24, **caractérisée en ce que**
la deuxième face est décalée par rapport à la première face.

26. Prothèse de genou selon l'une quelconque des revendications 17 à 25, **caractérisée en ce que**
la première face a une première zone et la deuxième face a une deuxième zone différente de la première zone.

27. Prothèse de genou selon l'une quelconque des revendications 17 à 26, **caractérisée en ce que**
le bord périphérique adjacent à la première des faces comprend un premier côté, un deuxième côté opposé au premier côté, une première extrémité, une deuxième extrémité opposée à la première extrémité, une première dimension D définie par la première extrémité et la deuxième extrémité, et une deuxième dimension F définie par le premier côté et le deuxième côté, la dimension F étant d'environ 0,25 à environ 1,5 de la dimension D.

28. Prothèse de genou selon l'une quelconque des revendications précédentes, **caractérisée en ce que**
le corps (802 ; 902) comprend une première partie (804 ; 904) et une deuxième partie (806 ; 906) ;
la première partie (804 ; 904) ayant un premier bord périphérique (808 ; 908) et une première face (810 ; 910) ;
la deuxième partie (806 ; 906) ayant un deuxième bord périphérique (812 ; 912) et une deuxième face (814 ; 914) ; et
le deuxième bord périphérique (812 ; 912) s'étendant radialement vers l'extérieur du premier bord périphérique (808 ; 908) pour former un épaulement (816 ; 916) entre la première partie (804 ; 904) et la deuxième partie (806 ; 906).

29. Prothèse de genou selon la revendication 28, **caractérisée en ce que**
le premier bord périphérique comprend un premier côté, un deuxième côté opposé au premier côté, une première extrémité, une deuxième extrémité opposée à la première extrémité, une première dimension D définie par la première extrémité et la deuxième extrémité, et une deuxième dimension F définie par le premier côté et le deuxième côté, la dimension F étant d'environ 0,25 à environ 1,5 de la dimension D.

30. Prothèse de genou selon l'une quelconque des revendications 19, 20 et/ou 28 à 29, **caractérisée en ce que**
la première partie est substantiellement en forme de haricot, comprenant une première encoche.

31. Prothèse de genou selon l'une quelconque des revendications 19, 20 et/ou 28 à 30, **caractérisée en ce que**
la deuxième partie est substantiellement en forme de haricot, comprenant une deuxième encoche.

32. Prothèse de genou selon la revendication 31, **caractérisée en ce que**
la deuxième encoche s'étend radialement vers l'extérieur de la première encoche.

33. Prothèse de genou selon l'une quelconque des revendications 19, 20 et/ou 28 à 32, **caractérisée en ce que**
la première partie comprend une première zone A1 et la deuxième partie comprend une deuxième zone A2, différente de la première zone A1.

34. Prothèse de genou selon la revendication 33, **caractérisée en ce que**
la première zone A1 est inférieure à la deuxième zone A2.

35. Prothèse de genou selon l'une quelconque des revendications 19, 20 et/ou 28 à 34, **caractérisée en ce que**
le premier bord périphérique comprend une première encoche et le deuxième bord périphérique comprend une deuxième encoche alignée avec la première encoche.

36. Prothèse de genou selon l'une quelconque des revendications 19, 20 et/ou 28 à 34, **caractérisée en ce que**
seul le premier bord périphérique comprend une encoche.

37. Prothèse de genou selon l'une quelconque des revendications précédentes, **caractérisée en ce que**
le corps (1002 ; 1102 ; 1202) est un corps en deux éléments ;
un premier élément (1004 ; 1104 ; 1204) étant un élément tibial comprenant une surface tibiale ;
un deuxième élément (1006 ; 1106 ; 1206) étant un élément fémoral comprenant une surface fémorale ;
le premier élément (1004 ; 1104 ; 1204) et le deuxième élément (1006 ; 1106 ; 1206) pouvant être mis en prise l'un avec l'autre et être séparés.

38. Prothèse de genou selon l'une quelconque des revendications 11 à 16 ou 37, **caractérisée en ce que**
les premier et deuxième éléments sont interconnectés par une clavette en prise dans une rainure.

39. Prothèse de genou selon l'une quelconque des revendications 11 à 16 ou 38, **caractérisée en ce que**
la clavette et la rainure sont substantiellement linéaires.

40. Prothèse de genou selon l'une quelconque des revendications 11 à 16 ou 38, **caractérisée en ce que**
la clavette et la rainure sont substantiellement arquées.

41. Prothèse de genou selon l'une quelconque des revendications 11 à 16 ou 37 à 40, **caractérisée en ce que**
les premier et deuxième éléments ont chacun une surface d'interface.

42. Prothèse de genou selon l'une quelconque des revendications 11 à 16 ou 41, **caractérisée en ce que**
chaque surface d'interface est substantiellement plane.

43. Prothèse de genou selon l'une quelconque des revendications 11 à 16, ou 41, **caractérisée en ce que**
chaque surface d'interface est substantiellement arquée.

44. Prothèse de genou selon l'une quelconque des revendications 11 à 16 ou 37 à 43, **caractérisée par**
un premier côté, un deuxième côté opposé au premier côté, une première extrémité, une deuxième extrémité opposée à la première extrémité, une première dimension D définie par la première extrémité et la deuxième extrémité, et une deuxième dimension F définie par le premier côté et le deuxième côté, la dimension F étant d'environ 0,25 à environ 1,5 de la dimension D.

45. Prothèse de genou selon l'une quelconque des revendications 10 à 16 ou 37 à 44, **caractérisée en ce que**
la surface fémorale comprend un rayon antérieur à postérieur RA et la première dimension D, D étant d'environ 0,6 RA à environ 1,4 RA.

46. Prothèse de genou selon l'une quelconque des revendications 12 à 16 ou 45, **caractérisée en ce que**
le rayon antérieur à postérieur RA est égal à la première dimension D.

47. Prothèse de genou selon l'une quelconque des revendications 37 à 46, **caractérisée par**
le premier élément ayant une première encoche et le deuxième élément ayant une deuxième encoche alignée avec la première encoche.
